# EUROPÄISCHE PATENTANMELDUNG

(11) **EP 4 382 088 A1**
(43) Veröffentlichungstag der Anmeldung: **12.06.2024**
(21) Anmeldenummer: 22212048.7
(22) Anmeldetag: 07.12.2022
(51) Int. Cl.: A61K 8/06, A61K 8/27, A61K 8/29, A61K 8/72, A61K 8/92, A61Q 17/04

(54) **EMULSIONEN, KOSMETISCHE ZUSAMMENSETZUNGEN UND SONNENSCHUTZMITTEL**

(71) Anmelder: Lignovations GmbH, 3430 Tulln an der Donau (AT)
(72) Erfinder: BEISL, Stefan, 3430 Tulln an der Donau (AT); MILTNER, Angela, 3430 Tulln an der Donau (AT); MILTNER, Martin, 3430 Tulln an der Donau (AT); TIBO, Victor, 3430 Tulln an der Donau (AT); TOMASICH, Julia, 3430 Tulln an der Donau (AT)
(74) Vertreter: SONN Patentanwälte GmbH & Co KG

(57) **Zusammenfassung**

Die Erfindung betrifft eine Emulsion sowie ein Sonnenschutzmittel enthaltend Ligninpartikel, wobei die Ligninpartikel einen mittleren hydrodynamischen Durchmesser von weniger als 800 nm und vorzugsweise einen Polydispersitätsindex von weniger als 0,50 aufweisen. Die Erfindung betrifft weiters eine kosmetische Zusammensetzung enthaltend die Emulsion.

## Beschreibung

Das Gebiet der vorliegenden Erfindung ist das der Emulsionen, kosmetischen Zusammensetzungen und Sonnenschutzmittel.

Unter einer Emulsion wird typischerweise ein fein verteiltes Gemisch zweier normalerweise nicht mischbarer Flüssigkeiten, beispielsweise einer Wasser- und einer Fettphase, bezeichnet. Emulsionen werden in einer Vielzahl unterschiedlicher Bereiche angewendet.

Emulsionen sind typischerweise thermodynamisch instabil. Die disperse Phase ist bestrebt, sich durch Koaleszenz zu größeren Bereichen zu vereinigen - dabei wird die Grenzflächenenergie zwischen den beiden Phasen verringert. Durch Emulgatoren, welche sich an den Grenzflächen zwischen den Phasen anreichern, lässt sich die Grenzflächenspannung senken. Dadurch kann eine höhere Koaleszenzstabilität erreicht werden. Es besteht allerdings ein Bedarf, stabilere Emulsionen, insbesondere mit höherer Temperatur- und Lagerstabilität zu bilden.

Einen besonders hohen Stellenwert haben Emulsionen im Bereich der Kosmetika bzw in Zusammensetzungen zum Auftragen auf die Haut, zB in Hautcremen. Durch die Wahl der Bestandteile und Mengen der Fett- und Wasserphase lassen sich vielfältige Texturen mit unterschiedlicher Viskosität bereitstellen. Zudem können eine Vielzahl unterschiedlicher Stoffe in Emulsionen eingearbeitet werden, z.B. Wirkstoffe. Aufgrund der weiten Verbreitung von Emulsionen in diesem Bereich besteht ein besonders großer Bedarf an Emulsionen, die sich gut auf der Haut verteilen lassen und die ein gutes Hautgefühl geben.

Ein besonders bedeutsamer Bereich in der Kosmetik sind Sonnenschutzmittel, die nicht ausschließlich, aber ebenfalls häufig in Form von Emulsionen bereitgestellt werden. Sonnenschutzmittel werden typischerweise auf die Haut aufgetragen, um die negativen Wirkungen der Sonnenstrahlung (wie Sonnenbrand mit Hautrötung, Blasenbildung, Hautalterung) zu mindern oder zu verhindern. Das sich ungeschützte Aussetzen intensiver Sonneneinstrahlung führt zu einem steigenden Hautkrebsrisiko. Die UV-Strahlen des Sonnenlichtes gelten als Hauptursache für lichtbedingte Hautschäden, weshalb Sonnenschutzmittel typischerweise UV-Filter enthalten, auch als Sonnenschutzfilter oder Lichtschutzmittel bezeichnet. Als UV-Filter werden typischer Stoffe bezeichnet, die ausschließlich oder überwiegend dazu bestimmt sind, die Haut durch Absorption, Reflexion oder Streuung bestimmter UV-Strahlung gegen bestimmte UV-Strahlung zu schützen. Typischerweise unterscheidet man organische (chemische) und anorganische (mineralische bzw physikalische) Filter. Konventionelle UV-Filter können negative Auswirkungen auf Menschen und Umwelt haben. Dazu zählen hormonelle und krebsauslösende Wirkungen im Körper bis hin zu Anreicherung in Umwelt und Tieren, was beispielsweise zur Korallenbleiche führen kann. Es besteht daher ein dringender Bedarf an Möglichkeiten, die Menge von UV-Filtern in Sonnenschutzmitteln zu reduzieren.

Es ist eine Aufgabe der vorliegenden Erfindung, zumindest einige der genannten Nachteile des Standes der Technik zu beseitigen bzw zu lindern. Insbesondere liegt eine Aufgabe der Erfindung darin, stabilere Emulsionen bereitzustellen. Eine weitere Aufgabe der Erfindung liegt darin, Emulsionen bereitzustellen, die verbesserte Eigenschaften für die Anwendung auf der Haut haben, insbesondere eine bessere Verteilbarkeit und/oder ein besseres Hautgefühl. Eine weitere Aufgabe besteht darin, Sonnenschutzmittel mit hohem UV-Schutz trotz geringerer Mengen an UV-Filtern bereitzustellen.

Diese Aufgaben werden gelöst durch eine Emulsion, eine kosmetische Zusammensetzung sowie ein Sonnenschutzmittel wie definiert in den unabhängigen Ansprüchen.

In einem ersten Aspekt betrifft die Erfindung eine Emulsion enthaltend Ligninpartikel, wobei die Ligninpartikel einen mittleren hydrodynamischen Durchmesser von weniger als 800 nm und vorzugsweise einen Polydispersitätsindex von weniger als 0,50 aufweisen.

In einem weiteren Aspekt stellt die Erfindung eine kosmetische Zusammensetzung enthaltend die erfindungsgemäße Emulsion bereit.

Ein weiterer Aspekt der Erfindung betrifft ein Sonnenschutzmittel enthaltend mindestens einen UV-Filter und Ligninpartikel, wobei die Ligninpartikel einen mittleren hydrodynamischen Durchmesser von weniger als 800 nm und vorzugsweise einen Polydispersitätsindex von weniger als 0,50 aufweisen.

Sämtliche Aspekte der Erfindung werden in der Folge gemeinsam beschrieben. Alle in Bezug auf die erfindungsgemäße Emulsion beschriebenen Ausführungsformen, z.B. in Bezug auf die Größe und Größenverteilung der Ligninpartikel, sind auch im Zusammenhang mit der erfindungsgemäßen kosmetischen Zusammensetzung sowie mit dem erfindungsgemäßen Sonnenschutzmittel bevorzugt und umgekehrt. Vorzugsweise weist das erfindungsgemäße Sonnenschutzmittel die erfindungsgemäße Emulsion auf.

Lignine sind feste Biopolymere bestehend aus phenolischen Makromolekülen, die in die pflanzliche Zellwand eingelagert sind. In Pflanzen sind Lignine vor allem für die Festigkeit des pflanzlichen Gewebes verantwortlich. Bei der Herstellung von Zellulose oder Papier aus pflanzlichem Material wird der feste Zellwandbestandteil Lignin durch verschiedene Verfahren (z.B. Sulfitverfahren, Kraft-Aufschluss, Organosolv Verfahren, enzymatische Verfahren) von der Zellulose getrennt.

Lignin ist ein in hohem Maße unregelmäßig verzweigter polyphenolischer Polyether, der aus den primären Monolignolen, p-Coumarylalkohol, Coniferylalkohol und Sinapylalkohol besteht, die über aromatische und aliphatische Etherbindungen verknüpft sind. Grob können drei verschiedene Typen von Ligninen unterschieden werden: Weichholz-Lignine sind nahezu ausschließlich aus Coniferylalkohol aufgebaut, Hartholz-Lignine aus Coniferyl- sowie Sinapylalkohol und Gras-Lignine aus allen drei Typen. Die hohe Komplexität und Inhomogenität der Ligninstruktur wird in vielen Fällen durch die derzeit angewandten Vorbehandlungs-Technologien sogar noch weiter gesteigert und führt zu zusätzlichen Herausforderungen für die Weiterverarbeitung und Verwertung des Lignins. Im Vergleich zu anderen Vorbehandlungs-Technologien wird das Lignin mit dem im vorliegenden Fall verwendeten Organosolv-Verfahren in relativ reiner, niedermolekularer Form aus der Biomasse extrahiert. Dieses Lignin zeigt ein Minimum an Kohlehydrat- und mineralischen Verunreinigungen und erleichtert Anwendungen des Lignins von höherem Wert als der Wärme- und Energieerzeugung.

Ein Ansatz zur Überwindung dieser hohen Komplexität und Inhomogenität besteht in der Produktion und Anwendung von nano- oder mikrostrukturiertem Lignin. Nanostrukturierte Materialien, insbesondere im Bereich von unter 1 µm, bieten einzigartige Eigenschaften aufgrund ihrer erhöhten spezifischen Oberfläche, wobei ihre wesentlichen chemischen und physikalischen Interaktionen durch die Oberflächeneigenschaften bestimmt werden. Folglich kann ein nano- und mikrostrukturiertes Material deutlich andere Eigenschaften aufweisen, als ein größer dimensioniertes Material derselben Zusammensetzung. Daher hat die Herstellung von Ligninpartikeln in diesem Größenbereich und anderen Nano- und Mikrostrukturen während der letzten Jahre das Interesse unter Forschern geweckt.

Lignin-Nano- und Mikropartikel finden diverse potenzielle Anwendungen, die von verbesserten mechanischen Eigenschaften von Polymer-Nanokompositen, bakteriziden und antioxidativen Eigenschaften und Imprägnationen, bis zu Arzneistoffträgern für hydrophobe und hydrophile Substanzen reichen.

Ligninpartikel, insbesondere mit einer Partikelgröße von weniger als 1 µm, sowie Verfahren zu deren Herstellung sind beispielsweise aus der WO 2018/115516 A1, der WO 2020/000008 A1 und aus Beisl et al. (Molecules 23 (2018), 633-646) bekannt. Im Zusammenhang mit der vorliegenden Erfindung werden vorzugsweise kolloidale Lignin-Partikel eingesetzt, insbesondere wie beschrieben in der WO 2018/115516 A1, der WO 2020/000008 A1 und/oder in Beisl et al. (Molecules 23 (2018), 633-646).

Lignin bzw Ligninpartikel mit verschiedenen Anwendungen, unter anderem auch im Zusammenhang mit Sonnenschutzmitteln, sind außerdem beschrieben in Lee et al. ("Preparation and application of light-colored lignin nanoparticles for broad-spectrum sunscreens." Polymers 12.3 (2020): 699), Qian et al. ("Lignin: a nature-inspired sun blocker for broad-spectrum sunscreens." Green Chemistry 17.1 (2015): 320-324), Widsten et al. ("Natural sunscreens based on nanoparticles of modified kraft lignin (Cat-Lignin)." ACS Omega 5.22 (2020): 13438-13446), Widsten ("Ligninbased sunscreens - State-of-the-art, prospects and challenges." Cosmetics 7.4 (2020): 85), Sadeghifar ("Lignin as A Natural Sunscreen-An Overview." Biomedical Journal of Scientific & Technical Research 27.1 (2020): 20389-20391), Lee et al. ("Lignin for white natural sunscreens." International Journal of Biological Macromolecules 122 (2019): 549-554), Qian et al. ("Fabrication of uniform lignin colloidal spheres for developing natural broad-spectrum sunscreens with high sun protection factor." Industrial Crops and Products 101 (2017) : 54-60) und Li et al. ("Preparation of organic acid lignin submicrometer particle as a natural broad-spectrum photo-protection agent." International Journal of Biological Macromolecules 132 (2019): 836-843). Lignin bzw Ligninpartikel mit unterschiedlichen Anwendungen sind außerdem beschrieben in WO 2020/209907 A1, WO 2020/007885 A1, CN 113101235 A, US 10,682,304 B1, CN 106361591 A, CN 113855809 A und CN 113332170 A.

Im Rahmen der vorliegenden Erfindung hat sich nun überraschenderweise gezeigt, dass kolloidale Lignin-Partikel besonders vorteilhafte Eigenschaften in Emulsionen aufweisen können. Unter anderem haben sich Emulsionen enthaltend solche Ligninpartikel als besonders stabil erwiesen. Außerdem haben sich besonders vorteilhafte Eigenschaften im Zusammenhang mit kosmetischen Mitteln bzw Mitteln zur Auftragung auf die Haut gezeigt, unter anderem eine gute Verteilbarkeit und ein gutes Hautgefühl. Diese speziellen Eigenschaften machen Ligninpartikel wie hierin beschrieben hervorragend geeignet für den Einsatz als funktioneller Inhaltsstoff in kosmetischen Endprodukten. Diese vorteilhaften Eigenschaften stehen in einem engen Zusammenhang mit der Größe bzw Größenverteilung der Ligninpartikel.

Ohne an eine bestimmte Theorie gebunden sein zu wollen, vermuten die Erfinder, dass sich Ligninpartikel in Emulsionen zumindest teilweise an den Grenzflächen zwischen Wasser- und Fettphase anreichern und die Grenzflächenspannung der beiden nichtmischbaren Phasen reduzieren. Die Ligninpartikel können also als Emulgatoren wirken. Dabei hat die Größe bzw Größenverteilung der Ligninpartikel einen entscheidenden Einfluss auf die Wirkung als Emulgator. So hat sich herausgestellt, dass ein mittlerer hydrodynamischer Durchmesser von weniger als 800 nm, insbesondere von weniger als 500 nm besonders vorteilhaft für die Herstellung von Emulsionen ist. Zusätzlich hat sich gezeigt, dass besondere Vorteile mit einer engen Partikelgrößenverteilung (d.h. eine geringe Polydispersität) einhergehen. Ohne an eine bestimmte Theorie gebunden sein zu wollen, vermuten die Erfinder, dass eine geringe Variabilität in der Größe der Ligninpartikel dazu führt, dass sich eine gleichmäßigere Schicht von Ligninpartikel an den Grenzflächen zwischen Wasser- und Fettphase bilden kann. Dies kann die Stabilität, unter anderem auch die Temperatur- und/oder Lagerstabilität, der Emulsion zusätzlich erhöhen. Darüber hinaus vermuten die Erfinder, dass die homogene Größenverteilung der Ligninpartikel an den Grenzflächen zu homogeneren Emulsionen führt, was ebenfalls zu einer besseren Stabilität und/oder zu vorteilhaften Eigenschaften bei Anwendungen auf der Haut (etwa gute Verteilbarkeit und gutes Hautgefühl) führen kann. Die Anreicherung der Ligninpartikel an den Grenzflächen zwischen Wasser- und Fettphase wurde von den Erfindern schließlich auch experimentell durch Fluoreszenzmikroskopie bestätigt (siehe Beispiel 3).

Weiters hat sich im Rahmen der Erfindung herausgestellt, dass Ligninpartikel mit den beschriebenen Partikelgrößen bzw -verteilungen besondere Vorteile im Zusammenhang mit Sonnenschutzmitteln aufweisen. So wurde eine starke Erhöhung des Lichtschutzfaktors (im Folgenden "SPF", Sun Protection Factor) beobachtet, wenn derartige Ligninpartikel zu Sonnenschutzmitteln hinzugefügt wurden. Die Ligninpartikel haben dabei für sich genommen keine starke Wirkung als UV-Filter, sondern sie verstärken die UV-Filterwirkung von im Sonnenschutzmittel vorliegenden UV-Filtern. Dieser "SPF-Booster" Effekt hängt nicht nur eng mit der Größe bzw Größenverteilung der Ligninpartikel zusammen, sondern zeigt überraschenderweise eine starke Abhängigkeit von der Konzentration der Ligninpartikel. So wurde beobachtet, dass der "SPF-Booster" Effekt bei geringen Ligninkonzentrationen nur moderat ausgeprägt ist, ab einer Konzentrationsschwelle zwischen 1,7 und 2,5 Gew.-% aber plötzlich sehr stark auftritt.

Die beschriebenen Vorteile im Zusammenhang mit Emulsionen und der "SPF-Booster"-Effekt sind grundsätzlich unabhängig voneinander. Die Ligninpartikel können vorteilhafterweise in Emulsionen ohne UV-Filter eingesetzt werden, zB in Kosmetika ohne UV-Schutz oder in Emulsionen auf anderen technischen Gebieten. Genauso kann der "SPF-Booster" Effekt in Sonnenschutzmitteln genutzt werden, welche keine Emulsion sind. Besonders vorteilhaft ist es aber, die vorteilhaften Effekte zu kombinieren und ein Sonnenschutzmittel enthaltend eine Emulsion wie oben beschrieben vorzusehen. Einerseits nutzen die Sonnenschutzmittel so den "SPF-Booster"-Effekt und weisen daher einen höheren SPF-Faktor auf bzw kommen mit geringeren Mengen von UV-Filtern aus. Andererseits weisen solche Sonnenschutzmittel die vorteilhaften Effekte in Bezug auf die Stabilität und Verteilbarkeit / Hautgefühl der Emulsion auf.

Wie oben erwähnt, hat sich im Rahmen der Erfindung gezeigt, dass die Größe und Größenverteilung der Ligninpartikel einen entscheidenden Einfluss auf die Eigenschaften der Emulsionen bzw der Sonnenschutzmittel hat. Vorzugsweise weisen die Ligninpartikel daher einen mittleren hydrodynamischen Durchmesser von weniger als 750 nm, bevorzugt weniger als 700 nm, mehr bevorzugt weniger als 650 nm, mehr bevorzugt weniger als 600 nm, mehr bevorzugt weniger als 550 nm, mehr bevorzugt weniger als 500 nm, auf. Es ist besonders bevorzugt, wenn die Ligninpartikel einen mittleren hydrodynamischen Durchmesser von 1 bis 800 nm, bevorzugt von 2 bis 750 nm, mehr bevorzugt von 3 bis 700 nm, mehr bevorzugt von 4 bis 650 nm, mehr bevorzugt von 6 bis 600 nm, mehr bevorzugt von 8 bis 575 nm, mehr bevorzugt von 10 bis 550 nm, mehr bevorzugt von 15 bis 525 nm, noch mehr bevorzugt von 20 bis 500 nm aufweisen. In Reihenfolge zunehmender Präferenz beträgt der mittlere hydrodynamische Durchmesser der Ligninpartikel mindestens 1, 2, 4, 6, 8, 10, 15, 20, 25, 30, 35, 40, 45, 50 nm. In Reihenfolge zunehmender Präferenz beträgt der mittlere hydrodynamische Durchmesser der Ligninpartikel nicht mehr als 800, 750, 700, 650, 600, 550, 500 nm.

Der mittlere hydrodynamische Durchmesser wird vorzugsweise bestimmt durch dynamische Lichtstreuung (DLS), vorzugsweise nach ISO 22412:2017, beispielsweise mit einem Anton Paar Litesizer 500.

Es hat sich besonders überraschend herausgestellt, dass eine enge Größenverteilung, d.h. eine geringe Polydispersität, der Ligninpartikel vorteilhaft im Zusammenhang mit der erfindungsgemäßen Emulsion bzw dem erfindungsgemäßen Sonnenschutzmittel ist. Wie oben näher beschrieben, vermuten die Erfinder, dass dies die Bildung einer gleichmäßigeren Schicht von Ligninpartikeln an den Grenzflächen zwischen Wasser- und Fettphase ermöglichen und so zu stabileren und homogeneren Emulsionen führen kann. Dies kann wiederum mit vorteilhaften Eigenschaften bei Anwendungen auf der Haut einhergehen, wie etwa eine gute Verteilbarkeit und ein besonders gutes Hautgefühl.

Ein Maß für die Breite der Partikelgrößenverteilung ist der Polydispersitätsindex. Es ist daher bevorzugt, wenn die Ligninpartikel einen Polydispersitätsindex von weniger als 0,50, bevorzugt weniger als 0,45, mehr bevorzugt weniger als 0,40, mehr bevorzugt weniger als 0,35, mehr bevorzugt weniger als 0,30, mehr bevorzugt weniger als 0,25, mehr bevorzugt weniger als 0,20. Vorzugsweise weisen die Ligninpartikel einen Polydispersitätsindex von 0,01 bis 0,50, bevorzugt von 0,05 bis 0,40, mehr bevorzugt von 0,10 bis 0,30, auf.

Der Polydispersitätsindex wird vorzugsweise ebenfalls bestimmt durch dynamische Lichtstreuung (DLS), vorzugsweise nach ISO 22412:2017, beispielsweise mit einem Anton Paar Litesizer 500.

Im Rahmen der Erfindung ist es bevorzugt, wenn die Konzentration der Ligninpartikel in der Emulsion bzw im Sonnenschutzmittel mindestens 0,1 Gew.-%, bevorzugt mindestens 0,25 Gew.-%, mehr bevorzugt mindestens 0,5 Gew.-%, mehr bevorzugt mindestens 0,75 Gew.-%, mehr bevorzugt mindestens 1,0 Gew.-%, mehr bevorzugt mindestens 1,25 Gew.-%, mehr bevorzugt mindestens 1,5 Gew.-%, mehr bevorzugt mindestens 1,7 Gew.-%, mehr bevorzugt mindestens 1,75 Gew.-%, mehr bevorzugt mindestens 1,8 Gew.-%, mehr bevorzugt mindestens 1,85 Gew.-%, mehr bevorzugt mindestens 1,9 Gew.-%, mehr bevorzugt mindestens 1,95 Gew.-%, mehr bevorzugt mindestens 2,0 Gew.-%, mehr bevorzugt mindestens 2,05 Gew.-%, mehr bevorzugt mindestens 2,1 Gew.-%, mehr bevorzugt mindestens 2,15 Gew.-%, mehr bevorzugt mindestens 2,2 Gew.-%, mehr bevorzugt mindestens 2,25 Gew.-%, mehr bevorzugt mindestens 2,3 Gew.-%, mehr bevorzugt mindestens 2,5 Gew.-%, mehr bevorzugt mindestens 2,7 Gew.-%, noch mehr bevorzugt mindestens 3,0 Gew.-% beträgt. Im Zusammenhang mit den erfindungsgemäßen Emulsionen ermöglicht es dies, dass ausreichend Ligninpartikel vorhanden sind, um sich an den Grenzflächen der Phasen anzureichen und diese zu stabilisieren. Höhere Konzentrationen der Ligninpartikel können die Bildung noch stabilerer und vorteilhafterer Emulsionen ermöglichen. Wie oben erwähnt, hat sich insbesondere bei den erfindungsgemäßen Sonnenschutzmitteln gezeigt, dass es ab einer Konzentration von zwischen 1,7 und 2,5 Gew.-% zu einem sprunghaften Anstieg des "SPF-Booster" Effekts kommt.

Allerdings ist es auch vorteilhaft, wenn die Konzentration der Ligninpartikel nicht zu hoch ist. Bei sehr hohen Konzentrationen kann die Viskosität der Zusammensetzungen ansteigen, was mit einer Verschlechterung des Hautgefühls einhergehen kann. Es ist daher bevorzugt, wenn die Konzentration der Ligninpartikel in der Emulsion bzw im Sonnenschutzmittel weniger als 40 Gew.-%, bevorzugt weniger als 30 Gew.-%, mehr bevorzugt weniger als 20 Gew.-%, mehr bevorzugt weniger als 15 Gew.-%, mehr bevorzugt weniger als 10 Gew.-%, mehr bevorzugt weniger als 8 Gew.-%, mehr bevorzugt weniger als 7 Gew.-%, noch mehr bevorzugt weniger als 6 Gew.-% beträgt. Vorzugsweise liegt die Konzentration im Bereich von 0,1 bis 50 Gew.-%, bevorzugt 0,25 bis 45 Gew.-%, mehr bevorzugt 0,5 bis 40 Gew.-%, mehr bevorzugt von 0,75 bis 37 Gew.-%, mehr bevorzugt von 1,0 bis 34 Gew.-%, mehr bevorzugt von 1,25 bis 32 Gew.-%, mehr bevorzugt von 1,5 bis 32 Gew.-%, mehr bevorzugt von 1,7 bis 30 Gew.-%, mehr bevorzugt von 1,75 bis 28 Gew.-%, mehr bevorzugt von 1,8 bis 26 Gew.-%, mehr bevorzugt von 1,85 bis 24 Gew.-%, mehr bevorzugt von 1,9 bis 22 Gew.-%, mehr bevorzugt von 1,95 bis 20 Gew.-%, mehr bevorzugt von 2,0 bis 18 Gew.-%, mehr bevorzugt von 2,05 bis 16 Gew.-%, mehr bevorzugt von 2,1 bis 14 Gew.-%, mehr bevorzugt von 2,15 bis 12 Gew.-%, mehr bevorzugt von 2,2 bis 10 Gew.-%, mehr bevorzugt von 2,25 bis 9 Gew.-%, mehr bevorzugt von 2,3 bis 8 Gew.-%, mehr bevorzugt von 2,5 bis 7 Gew.-%, mehr bevorzugt von 2,7 bis 6 Gew.-%, noch mehr bevorzugt von 3,0 bis 5 Gew.-%. Besonders bevorzugt ist ein Bereich von 0,5 bis 10 Gew.%, bevorzugt 1,7 bis 7 Gew.-%, noch mehr bevorzugt 1,9 bis 6,5 Gew.-%, noch mehr bevorzugt 2,1 bis 6,0 Gew.-%, noch mehr bevorzugt 2,3 bis 5,5 Gew.-%, insbesondere bevorzugt 2,5 bis 5 Gew.-%.

Es hat sich gezeigt, dass die Ligninpartikel wie oben beschrieben sowohl für die Bildung von Öl-in-Wasser Emulsionen als auch von Wasser-in-Öl Emulsionen geeignet ist. In einer bevorzugten Ausführungsform handelt es sich daher bei der erfindungsgemäßen Emulsion um eine Öl-in-Wasser Emulsion. In einer weiteren bevorzugten Ausführungsform handelt es sich bei der erfindungsgemäßen Emulsion um eine Wasser-in-Öl Emulsion. Besonders bevorzugt sind allerdings Öl-in-Wasser Emulsionen.

Es hat sich weiters gezeigt, dass die Ligninpartikel wie oben beschrieben eine große Bandbreite von Verhältnissen zwischen der Öl- und der Wasserphase stabilisieren können. So ermöglichen es die Ligninpartikel etwa, stabile Emulsionen mit besonders hohem Wasseranteil aber auch alternativ dazu mit besonders hohem Fettanteil bereitzustellen. Vorzugsweise weist die Emulsion eine Fettphase und eine Wasserphase auf und der Anteil der Wasserphase an der Emulsion beträgt mehr als 10 Gew.-%, bevorzugt mehr als 15 Gew.-%, mehr bevorzugt mehr als 20 Gew.-%, noch mehr bevorzugt mehr als 30 Gew.-%, noch mehr bevorzugt mehr als 40 Gew.-%, noch mehr bevorzugt mehr als 50 Gew.-%, noch mehr bevorzugt mehr als 60 Gew.-%, noch mehr bevorzugt mehr als 70 Gew.-%. Weiters ist es bevorzugt, wenn der Anteil der Wasserphase an der Emulsion weniger als 90 Gew.-%, bevorzugt weniger als 85 Gew.-%, mehr bevorzugt weniger als 80 Gew.-%, noch mehr bevorzugt weniger als 70 Gew.-%, noch mehr bevorzugt weniger als 60 Gew.-%, noch mehr bevorzugt weniger als 50 Gew.-%, noch mehr bevorzugt weniger als 40 Gew.-%, noch mehr bevorzugt weniger als 30 Gew.-% beträgt. Vorzugsweise beträgt der Anteil der Wasserphase an der Emulsion von 10 bis 90 Gew.-%, bevorzugt von 20 bis 80 Gew.-%, noch mehr bevorzugt von 25 bis 70 Gew.-%, noch mehr bevorzugt von 30 bis 60 Gew.-%. Weiters ist es bevorzugt, wenn der Anteil der Fettphase an der Emulsion von 10 bis 90 Gew.-%, bevorzugt von 15 bis 80 Gew.-%, noch mehr bevorzugt von 20 bis 70 Gew.-%, noch mehr bevorzugt von 25 bis 60 Gew.-% beträgt.

Für die erfindungsgemäße Emulsionen können übliche Bestandteile für die Fettphase eingesetzt werden. Die Fachperson ist mit solchen Bestandteilen vertraut. Bevorzugt ist es, wenn die Fettphase ein Fett, vorzugsweise eine Butter, ein Wachs, ein hydriertes Pflanzenöl und/oder ein tierisches Fett, aufweist. Die Butter ist vorzugsweise ausgewählt aus Butyrospermum Parkii Butter, Mangifera Indica Seed Butter, Theobroma Cacao Seed Butter und/oder Theobroma grandiflorum Butter. Das Wachs ist vorzugsweise ausgewählt aus cera flava, Copernicia Cerifera Cera, Euphorbia Cerifera (Candelilla) Wax, Candelilla Cera, Rapswachs, Rhus Verniciflua (Peel) Cera und/oder Rosa Multiflora Flower Wax. Das hydrierte Pflanzenöl ist vorzugsweise ausgewählt aus Hydrogenated Ethylhexyl Olivate Hydrogenated Olive Oil Unsaponifiables, Hydrogenated Olive Oil Unsaponifiables und/oder Hydrogenated Ricinus Oil. Das tierische Fett ist vorzugsweise Adeps Lanae.

Weiters ist es bevorzugt, wenn die Fettphase ein Öl, vorzugsweise ein pflanzliches Öl und/oder ein synthetisches Öl, aufweist. Vorzugsweise ist das pflanzliche Öl ausgewählt aus Adansonia Digitata Seed Oil, Argania Spinosa Kernel Oil, Black Cumin seed oil, Borago Officinalis (Borage) Seed Oil, Brassica Oleracea (Broccoli) Seed Oil, Calendula officinalis, Calophyllum Inophyllum, Cannabis Sativa Seed Oil, Caprylic/Capric Triglyceride, Caryodendron Orinocense Seed Oil, Coffea Arabica Seed Oil, Elaeis Guineensis Kernel Oil, Glycine Soja Seed Oil, Helianthus annuus seed oil, Macadamia Ternifolia Seed Oil, Oenothera Biennis Seed (Evening Primrose) Oil, Olea Europaea Fruit Oil, Orbignya Oleifera (Babassu) Seed Oil, Oryza Sativa (Rice Germ) Oil, Persea Gratissima (Avocado) Oil, Persea Gratissima (Avocado) Oil, Prunus Amygdalus Dulcis Oil, Prunus Armeniaca (Apricot) Kernel Oil, Ricinus Communis, Rubus Idaeus Seed Oil, Sesamum Indicum (Sesame) Oil, Simmondsia Chinensis (Jojoba) Seed Oil, Simmondsia Chinesis Oil, Sulfated Castor Oil, Triticum Vulgare (Wheat Germ) Oil, Undecane, Tridecane, Vaccinium Macrocarpan (Cranberry) Seed Oil, Vitis Vinifera (Grape) Seed Oil, Cocos nucifera Oil und/oder Glycine soja Oil. Vorzugsweise ist das synthetische Öl ausgewählt aus Paraffinum Liquidum, C12-15 Alkyl Benzoate, LINOLEIC ACID, Butyloctyl Salicylate, Dimethicone, Cyclomethicone, Polysiloxane und/order Methicone.

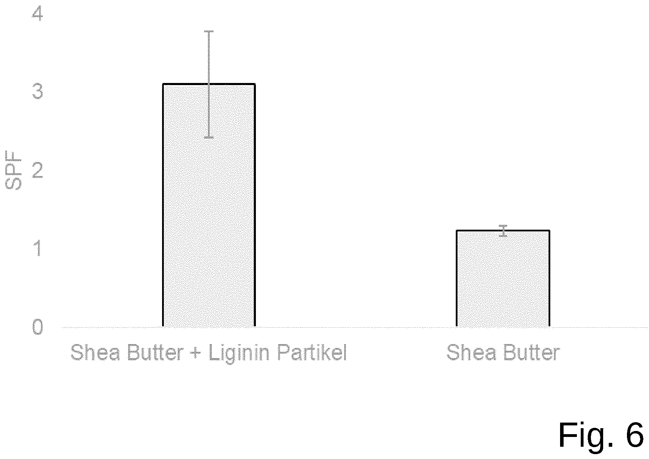

Im Rahmen der Erfindung hat sich allerdings gezeigt, dass die Auswahl bestimmter Öle und Fette mit besonderen Vorteilen einhergeht. So gibt es Öle und Fette, die zwar üblicherweise nicht eigentlich zum Zweck des UV-Schutzes eingesetzt werden, die aber eine natürliche UV-Absorption haben. In den meisten Fällen ist diese UV-Absorption zu niedrig, um einen wesentlichen praktischen Nutzen als UV-Schutz bereitzustellen. Im Rahmen der Erfindung hat sich aber überraschenderweise gezeigt, dass die Ligninpartikel wie hierin beschrieben den natürlichen UV-Schutz der Fette bzw Öle deutlich erhöhen können und so zu einem praktisch bedeutsamen UV-Schutz führen können. Dies wird zB in Beispiel 8 und Figur 6 gezeigt, worin eine Formulierung enthaltend Shea Butter für sich genommen nur einen geringen UV-Schutz hatte (SPF-Faktor 1.2), dieser aber in Anwesenheit von Ligninpartikel wie hierin beschrieben deutlich anstieg (SPF-Faktor 3.1). So kann ein UV-Schutz erreicht werden, ohne dass ein zusätzlicher UV-Filter eingesetzt werden muss.

Dieser Effekt ist sowohl im Zusammenhang mit der erfindungsgemäßen Emulsion als auch mit dem erfindungsgemäßen Sonnenschutzmittel (unabhängig davon, ob dieses eine Emulsion ist oder nicht) vorteilhaft. In Zusammensetzungen enthaltend keinen zusätzlichen UV-Filter ermöglicht es dieser Effekt, einen UV-Schutz bereitzustellen, ohne dass ein zusätzlicher Bestandteil erforderlich wäre. Dies ist insbesondere bei kosmetischen Zusammensetzungen vorteilhaft, bei denen zumindest ein gewisser UV-Schutz Hautschäden und Hautalterung vorbeugen kann, bei denen aber ein herkömmlicher UV-Filter mit möglichen negativen Auswirkungen auf Menschen und Umwelt vermieden werden soll. Aber auch im Zusammenhang mit Sonnenschutzmitteln enthaltend zusätzliche UV-Filter ist dieser Effekt höchst vorteilhaft, da die Menge der eingesetzten UV-Filter bei gleichbleibendem SPF-Faktor reduziert werden kann.

Um diese Effekte zu erreichen, sind Fette und/oder Öle geeignet, die bereits selbst einen SPF-Faktor in zumindest gewisser Höhe aufweisen (welchen die Ligninpartikel dann verstärken können). Der SPF-Faktor, wie hierin verwendet, wird vorzugsweise bestimmt nach der Norm DIN EN ISO 24443:2020-06, insbesondere wie in den nachfolgenden Beispielen, insbesondere Beispiel 8, beschrieben. Alternativ dazu kann die Bestimmung auch durchgeführt werden wie beschrieben in Kaur et al. ("In vitro sun protection factor determination of herbal oils used in cosmetics." Pharmacognosy Research 2.1 (2010): 22).

Vorzugsweise weist die Fettphase der Emulsion ein Fett und/oder ein Öl auf, wobei das Fett und/oder das Öl einen SPF-Faktor von mindestens 0,1, bevorzugt mindestens 0,2, mehr bevorzugt mindestens 0,3, mehr bevorzugt mindestens 0,4, mehr bevorzugt mindestens 0,5, mehr bevorzugt mindestens 0,7, mehr bevorzugt mindestens 1,0, mehr bevorzugt mindestens 2,0, aufweist. Besonders bevorzugt ist es, wenn der SPF-Faktor von 0,1 bis 20, bevorzugt von 0,2 bis 16, mehr bevorzugt 0,3 bis 14, mehr bevorzugt 0,4 bis 12, mehr bevorzugt 0,5 bis 11, mehr bevorzugt 0,6 bis 10, mehr bevorzugt 0,7 bis 9, mehr bevorzugt 0,8 bis 8, beträgt. In gleicher Weise ist es für das erfindungsgemäße Sonnenschutzmittel bevorzugt, wenn das Sonnenschutzmittel ein Fett und/oder ein Öl mit einem SPF-Faktor wie beschrieben aufweist.

Eine Reihe von Fetten und/oder Ölen mit geeigneter UV-Absorption, zB mit SPF-Faktoren im Bereich von 0,1 bis 20, sind beispielsweise beschrieben in Goswami et al. (Sch Acad J Pharm 2.6 (2013): 458-463) und Kaur et al. (Pharmacognosy Research 2.1 (2010): 22). Im Rahmen der Erfindung als besonders vorteilhaft herausgestellt haben sich Fette und/oder Öle ausgewählt aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl, Weizenkeimöl, Walnussöl, Baumwollsamenöl und/oder Mischungen davon; insbesondere Shea Butter. Es ist daher bevorzugt, wenn die Emulsion bzw das Sonnenschutzmittel zumindest ein solches Fett und/oder Öl aufweist.

Vorzugsweise beträgt die Konzentration des Fetts und/oder des Öls in der Emulsion bzw im Sonnenschutzmittel mehr als 10 Gew.-%, bevorzugt mehr als 15 Gew.-%, mehr bevorzugt mehr als 20 Gew.-%, noch mehr bevorzugt mehr als 30 Gew.-%, noch mehr bevorzugt mehr als 40 Gew.-%, noch mehr bevorzugt mehr als 50 Gew.-%, noch mehr bevorzugt mehr als 60 Gew.-%, noch mehr bevorzugt mehr als 70 Gew.-%. Vorzugsweise beträgt die Konzentration von 10 bis 90 Gew.-%, bevorzugt von 15 bis 80 Gew.-%, noch mehr bevorzugt von 20 bis 70 Gew.-%, noch mehr bevorzugt von 25 bis 60 Gew.-%.

Die Wasserphase der Emulsion kann mindestens einen Bestandteil ausgewählt aus Puffer, wasserlösliche Antioxidantien, wässrige Extrakte zur Farbgebung, Co-Emulgatoren, Konservierungsmittel, und/oder Verdicker aufweisen. Vorzugsweise weist die Wasserphase mindestens einen aktiven Wirkstoff auf.

Vorzugsweise beträgt der pH-Wert der Emulsion von 3 bis 8, bevorzugt von 3,5 bis 7,5, insbesondere von 4 bis 7.

Wie oben näher beschrieben, hat sich die Emulsion als besonders vorteilhaft im Zusammenhang mit kosmetischen Zusammensetzungen erwiesen. Sowohl die gute Stabilität der Emulsionen, insbesondere auch die Temperatur- und Lagerstabilität, sowie die gute Verteilbarkeit und das gute Hautgefühl das mit den oben beschriebenen Ausführungsformen der Emulsionen einhergehen, kommen bei kosmetischen Zusammensetzungen besonders zum Tragen. Die erfindungsgemäßen Emulsionen sind für eine Vielzahl unterschiedlicher kosmetischer Anwendungen geeignet. Bevorzugterweise handelt es sich bei der kosmetischen Zusammensetzung um eine BB (Blemish Balm) Cream, eine Tagescreme, eine Foundation, eine Mousse Formulation, einen Lippenstift, einen Body Tanner, eine Augenbrauen Pomade und/oder einen Concealer.

Im Zusammenhang mit dem erfindungsgemäßen Sonnenschutzmittel ist es bevorzugt, wenn die Konzentration des mindestens einen UV-Filters im Sonnenschutzmittel im Bereich von 1 bis 35 Gew.-%, bevorzugt von 1,5 bis 30 Gew.-%, insbesondere von 2,5 bis 25 Gew.-%, liegt. In Reihenfolge zunehmender Präferenz beträgt die Konzentration des mindestens einen UV-Filters weniger als 35, 30, 25, 20, 15, 10 Gew.-%. Durch den beschriebenen "SPF-Booster" Effekt können hohe Lichtschutzfaktoren mit niedrigen oder moderaten UV-Filter Konzentrationen erreicht werden.

Es hat sich gezeigt, dass ein "SPF-Booster" Effekt sowohl bei organischen als auch bei anorganischen UV-Filtern auftritt. Es können daher alle gängigen UV-Filter im erfindungsgemäßen Sonnenschutzmittel eingesetzt werden. Vorzugsweise ist der UV-Filter ausgewählt aus der Liste der in kosmetischen Mitteln zugelassenen UV-Filter gemäß Anhang VI Verordnung (EG) Nr. 1223/2009 des Europäischen Parlaments und des Rates vom 30. November 2009 über kosmetische Mittel (konsolidierter Text vom 06.10.2022, European Legislation Identifier (ELI): http://data.europa.eu/eli/reg/2009/1223/2022-10-06).

In einer bevorzugten Ausführungsform ist der mindestens eine UV-Filter ein anorganischer UV-Filter. Vorzugsweise ist der anorganische UV-Filter ausgewählt aus der Gruppe bestehend aus Zinkoxid und/oder Titanium Dioxid.

Wenn der mindestens eine UV-Filter ein anorganischer UV-Filter ist, hat es sich als besonders vorteilhaft erwiesen, wenn die Konzentration der Ligninpartikel nicht zu hoch ist. Höhere Konzentrationen führen in diesem Fall zu einer schlechteren Verteilbarkeit und einem schlechteren Hautgefühl. Die Erfinder vermuten, dass dies an einer höheren Viskosität liegt, wenn die anorganischen UV-Filter und Ligninpartikeln zusammen vorliegen. In diesem Fall ist es daher bevorzugt, wenn die Konzentration der Ligninpartikel im Sonnenschutzmittel nicht mehr als 7 Gew.-%, vorzugsweise nicht mehr als 6 Gew.-%, insbesondere nicht mehr als 5 Gew.-% beträgt.

In einer weiteren Ausführungsform ist der mindestens eine UV-Filter ein organischer UV-Filter.

Wenn der mindestens eine UV-Filter ein organischer UV-Filter ist, hat es sich herausgestellt, dass höhere Konzentrationen von Ligninpartikeln eingesetzt werden können, ohne dass sich die Verteilbarkeit oder das Hautgefühl verschlechtern. Die Erfinder vermuten, dass dies daran liegt, dass organische Filter in den erfindungsgemäßen Sonnenschutzmitteln einen weniger starken Beitrag zu einer hohen Viskosität liefern. In diesem Fall ist es vorteilhaft, wenn die Konzentration der Ligninpartikel mindestens 1,7 Gew.-%, bevorzugt mindestens 2,0 Gew.-%, noch mehr bevorzugt mindestens 2,3 Gew.-%, noch mehr bevorzugt mindestens 2,6 Gew.-%, noch mehr bevorzugt mindestens 2,9 Gew.-%, noch mehr bevorzugt mindestens 3,2 Gew.-%, noch mehr bevorzugt mindestens 3,5 Gew.-%, noch mehr bevorzugt mindestens 3,8 Gew.-%, noch mehr bevorzugt mindestens 4,1 Gew.-%, noch mehr bevorzugt mindestens 4,4 Gew.-%, noch mehr bevorzugt mindestens 4,7 Gew.-%, noch mehr bevorzugt mindestens 5,0 Gew.-%, noch mehr bevorzugt mindestens 5,3 Gew.-%, noch mehr bevorzugt mindestens 5,6 Gew.-%, noch mehr bevorzugt mindestens 5,9 Gew.-%, noch mehr bevorzugt mindestens 6,2 Gew.-%, noch mehr bevorzugt mindestens 6,5 Gew.-% beträgt. Es ist aber weiterhin bevorzugt, wenn die Konzentration nicht zu hoch ist. In Reihenfolge zunehmender Präferenz ist es vorteilhaft, wenn die Konzentration nicht mehr als 10 Gew.-%, bevorzugt nicht mehr als 9 Gew.-%, mehr bevorzugt nicht mehr als 8 Gew.-%, insbesondere nicht mehr als 7 Gew.-% beträgt.

Sämtliche gängige Anwendungsformen von Sonnenschutzmitteln sind im Rahmen der Erfindung geeignet. Die Fachperson ist mit solchen Anwendungsformen vertraut. Vorzugsweise ist das Sonnenschutzmittel eine Sonnencreme, eine Sonnenlotion, ein Sonnengel, ein Lippenbalsam, ein Spray, ein Öl, ein Tonic und/oder eine Milch. Besonders bevorzugt ist es, wenn das Sonnenschutzmittel die erfindungsgemäße Emulsion aufweist.

Sämtliche hierin genannten Parameter beziehen sich, wenn nicht anders gekennzeichnet, auf SATP-Bedingungen nach IUPAC ("Standard Ambient Temperature and Pressure"), insbesondere auf eine Temperatur von 25 °C und einen Druck von 101300 Pa.

Sämtliche Prozent-Angaben (%) hierin beziehen sich, wenn nicht anders gekennzeichnet, auf Gewichtsprozent.

Insbesondere betrifft die Erfindung die folgenden bevorzugten Ausführungsformen:
Ausführungsform 1. Emulsion enthaltend Ligninpartikel, wobei die Ligninpartikel einen mittleren hydrodynamischen Durchmesser von weniger als 800 nm aufweisen.
Ausführungsform 2. Emulsion gemäß Ausführungsform 1, wobei die Ligninpartikel einen mittleren hydrodynamischen Durchmesser von weniger als 750 nm, bevorzugt weniger als 700 nm, mehr bevorzugt weniger als 650 nm, mehr bevorzugt weniger als 600 nm, mehr bevorzugt weniger als 550 nm, mehr bevorzugt weniger als 500 nm aufweisen.
Ausführungsform 3. Die Emulsion gemäß Ausführungsform 1 oder 2, wobei die Ligninpartikel einen mittleren hydrodynamischen Durchmesser von 1 bis 800 nm, bevorzugt von 2 bis 750 nm, mehr bevorzugt von 3 bis 700 nm, mehr bevorzugt von 4 bis 650 nm, mehr bevorzugt von 6 bis 600 nm, mehr bevorzugt von 8 bis 575 nm, mehr bevorzugt von 10 bis 550 nm, mehr bevorzugt von 15 bis 525 nm, noch mehr bevorzugt von 20 bis 500 nm aufweisen.
Ausführungsform 4. Die Emulsion gemäß einer der vorhergehenden Ausführungsformen, wobei die Ligninpartikel einen Polydispersitätsindex von weniger als 0,50, bevorzugt weniger als 0,45, mehr bevorzugt weniger als 0,40, mehr bevorzugt weniger als 0,35, mehr bevorzugt weniger als 0,30, mehr bevorzugt weniger als 0,25, mehr bevorzugt weniger als 0,20.
Ausführungsform 5. Die Emulsion gemäß einer der vorhergehenden Ausführungsformen, wobei die Ligninpartikel einen Polydispersitätsindex von 0,01 bis 0,50, bevorzugt von 0,05 bis 0,40, mehr bevorzugt von 0,10 bis 0,30 aufweisen.
Ausführungsform 6. Die Emulsion gemäß einer der vorhergehenden Ausführungsformen, wobei die Konzentration der Ligninpartikel in der Emulsion mindestens 0,1 Gew.-%, bevorzugt mindestens 0,25 Gew.-%, mehr bevorzugt mindestens 0,5 Gew.-%, mehr bevorzugt mindestens 0,75 Gew.-%, mehr bevorzugt mindestens 1,0 Gew.-%, mehr bevorzugt mindestens 1,25 Gew.-%, mehr bevorzugt mindestens 1,5 Gew.-%, mehr bevorzugt mindestens 1,7 Gew.-%, mehr bevorzugt mindestens 1,75 Gew.-%, mehr bevorzugt mindestens 1,8 Gew.-%, mehr bevorzugt mindestens 1,85 Gew.-%, mehr bevorzugt mindestens 1,9 Gew.-%, mehr bevorzugt mindestens 1,95 Gew.-%, mehr bevorzugt mindestens 2,0 Gew.-%, mehr bevorzugt mindestens 2,05 Gew.-%, mehr bevorzugt mindestens 2,1 Gew.-%, mehr bevorzugt mindestens 2,15 Gew.-%, mehr bevorzugt mindestens 2,2 Gew.-%, mehr bevorzugt mindestens 2,25 Gew.-%, mehr bevorzugt mindestens 2,3 Gew.-%, mehr bevorzugt mindestens 2,5 Gew.-%, mehr bevorzugt mindestens 2,7 Gew.-%, noch mehr bevorzugt mindestens 3,0 Gew.-% beträgt.
Ausführungsform 7. Die Emulsion gemäß einer der vorhergehenden Ausführungsformen, wobei die Konzentration der Ligninpartikel in der Emulsion weniger als 40 Gew.-%, bevorzugt weniger als 30 Gew.-%, mehr bevorzugt weniger als 20 Gew.-%, mehr bevorzugt weniger als 15 Gew.-%, mehr bevorzugt weniger als 10 Gew.-%, mehr bevorzugt weniger als 8 Gew.-%, mehr bevorzugt weniger als 7 Gew.-%, noch mehr bevorzugt weniger als 6 Gew.-% beträgt.
Ausführungsform 8. Die Emulsion gemäß einer der vorhergehenden Ausführungsformen, wobei die Konzentration der Ligninpartikel in der Emulsion im Bereich von 0,1 bis 50 Gew.-%, bevorzugt 0,25 bis 45 Gew.-%, mehr bevorzugt 0,5 bis 40 Gew.-%, mehr bevorzugt von 0,75 bis 37 Gew.-%, mehr bevorzugt von 1,0 bis 34 Gew.-%, mehr bevorzugt von 1,25 bis 32 Gew.-%, mehr bevorzugt von 1,5 bis 32 Gew.-%, mehr bevorzugt von 1,7 bis 30 Gew.-%, mehr bevorzugt von 1,75 bis 28 Gew.-%, mehr bevorzugt von 1,8 bis 26 Gew.-%, mehr bevorzugt von 1,85 bis 24 Gew.-%, mehr bevorzugt von 1,9 bis 22 Gew.-%, mehr bevorzugt von 1,95 bis 20 Gew.-%, mehr bevorzugt von 2,0 bis 18 Gew.-%, mehr bevorzugt von 2,05 bis 16 Gew.-%, mehr bevorzugt von 2,1 bis 14 Gew.-%, mehr bevorzugt von 2,15 bis 12 Gew.-%, mehr bevorzugt von 2,2 bis 10 Gew.-%, mehr bevorzugt von 2,25 bis 9 Gew.-%, mehr bevorzugt von 2,3 bis 8 Gew.-%, mehr bevorzugt von 2,5 bis 7 Gew.-%, mehr bevorzugt von 2,7 bis 6 Gew.-%, noch mehr bevorzugt von 3,0 bis 5 Gew.-% beträgt.
Ausführungsform 9. Die Emulsion gemäß einer der vorhergehenden Ausführungsformen, wobei die Emulsion eine Öl-in-Wasser Emulsion ist.
Ausführungsform 10. Die Emulsion gemäß einer der vorhergehenden Ausführungsformen, wobei die Emulsion eine Wasser-in-Öl Emulsion ist.
Ausführungsform 11. Die Emulsion gemäß einer der vorhergehenden Ausführungsformen, wobei die Emulsion eine Fettphase und eine Wasserphase aufweist und wobei der Anteil der Wasserphase an der Emulsion mehr als 10 Gew.-%, bevorzugt mehr als 15 Gew.-%, mehr bevorzugt mehr als 20 Gew.-%, noch mehr bevorzugt mehr als 30 Gew.-%, noch mehr bevorzugt mehr als 40 Gew.-%, noch mehr bevorzugt mehr als 50 Gew.-%, noch mehr bevorzugt mehr als 60 Gew.-%, noch mehr bevorzugt mehr als 70 Gew.-% beträgt.
Ausführungsform 12. Die Emulsion gemäß einer der vorhergehenden Ausführungsformen, wobei die Emulsion eine Fettphase und eine Wasserphase aufweist und wobei der Anteil der Wasserphase an der Emulsion weniger als 90 Gew.-%, bevorzugt weniger als 85 Gew.-%, mehr bevorzugt weniger als 80 Gew.-%, noch mehr bevorzugt weniger als 70 Gew.-%, noch mehr bevorzugt weniger als 60 Gew.-%, noch mehr bevorzugt weniger als 50 Gew.-%, noch mehr bevorzugt weniger als 40 Gew.-%, noch mehr bevorzugt weniger als 30 Gew.-% beträgt.
Ausführungsform 13. Die Emulsion gemäß einer der vorhergehenden Ausführungsformen, wobei die Emulsion eine Fettphase und eine Wasserphase aufweist und wobei der Anteil der Wasserphase an der Emulsion von 10 bis 90 Gew.-%, bevorzugt von 20 bis 80 Gew.-%, noch mehr bevorzugt von 25 bis 70 Gew.-%, noch mehr bevorzugt von 30 bis 60 Gew.-% beträgt.
Ausführungsform 14. Die Emulsion gemäß einer der vorhergehenden Ausführungsformen, wobei die Emulsion eine Fettphase und eine Wasserphase aufweist und wobei der Anteil der Fettphase an der Emulsion von 10 bis 90 Gew.-%, bevorzugt von 15 bis 80 Gew.-%, noch mehr bevorzugt von 20 bis 70 Gew.-%, noch mehr bevorzugt von 25 bis 60 Gew.-% beträgt.
Ausführungsform 15. Die Emulsion gemäß einer der vorhergehenden Ausführungsformen, wobei die Emulsion eine Fettphase und eine Wasserphase aufweist und wobei die Fettphase ein Fett, vorzugsweise eine Butter, ein Wachs, ein hydriertes Pflanzenöl und/oder ein tierisches Fett; und/oder ein Öl, vorzugsweise ein pflanzliches Öl und/oder ein synthetisches Öl, aufweist.
Ausführungsform 16. Die Emulsion gemäß einer der vorhergehenden Ausführungsformen, wobei die Emulsion eine Fettphase und eine Wasserphase aufweist, wobei die Fettphase ein Fett und/oder ein Öl aufweist und wobei das Fett und/oder das Öl einen SPF-Faktor von mindestens 0,1, bevorzugt mindestens 0,2, mehr bevorzugt mindestens 0,3, mehr bevorzugt mindestens 0,4, mehr bevorzugt mindestens 0,5, mehr bevorzugt mindestens 0,7, mehr bevorzugt mindestens 1,0, mehr bevorzugt mindestens 2,0, aufweist.
Ausführungsform 17. Die Emulsion gemäß einer der vorhergehenden Ausführungsformen, wobei das Fett und/oder das Öl einen SPF-Faktor von 0,1 bis 20, bevorzugt von 0,2 bis 16, mehr bevorzugt 0,3 bis 14, mehr bevorzugt 0,4 bis 12, mehr bevorzugt 0,5 bis 11, mehr bevorzugt 0,6 bis 10, mehr bevorzugt 0,7 bis 9, mehr bevorzugt 0,8 bis 8, aufweist.
Ausführungsform 18. Die Emulsion gemäß einer der vorhergehenden Ausführungsformen, wobei das Fett und/oder das Öl ausgewählt ist aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl, Weizenkeimöl, Walnussöl, Baumwollsamenöl und/oder Mischungen davon; insbesondere Shea Butter. Ausführungsform 19. Die Emulsion gemäß einer der vorhergehenden Ausführungsformen, wobei die Emulsion ein Fett und/oder ein Öl ausgewählt aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl, Weizenkeimöl, Walnussöl, Baumwollsamenöl und/oder Mischungen davon aufweist, insbesondere Shea Butter.
Ausführungsform 20. Die Emulsion gemäß einer der vorhergehenden Ausführungsformen, wobei Konzentration des Fetts und/oder des Öls in der Emulsion mehr als 10 Gew.-%, bevorzugt mehr als 15 Gew.-%, mehr bevorzugt mehr als 20 Gew.-%, noch mehr bevorzugt mehr als 30 Gew.-%, noch mehr bevorzugt mehr als 40 Gew.-%, noch mehr bevorzugt mehr als 50 Gew.-%, noch mehr bevorzugt mehr als 60 Gew.-%, noch mehr bevorzugt mehr als 70 Gew.-% beträgt.
Ausführungsform 21. Die Emulsion gemäß einer der vorhergehenden Ausführungsformen, wobei Konzentration des Fetts und/oder des Öls in der Emulsion von 10 bis 90 Gew.-%, bevorzugt von 15 bis 80 Gew.-%, noch mehr bevorzugt von 20 bis 70 Gew.-%, noch mehr bevorzugt von 25 bis 60 Gew.-% beträgt.
Ausführungsform 22. Die Emulsion gemäß einer der vorhergehenden Ausführungsformen, wobei die Emulsion eine Fettphase und eine Wasserphase aufweist und wobei die Wasserphase mindestens einen Bestandteil ausgewählt aus Puffer, wasserlösliche Antioxidantien, wässrige Extrakte zur Farbgebung, Co-Emulgatoren, Konservierungsmittel, und/oder Verdicker aufweist.
Ausführungsform 23. Die Emulsion gemäß einer der vorhergehenden Ausführungsformen, wobei die Emulsion eine Fettphase und eine Wasserphase aufweist und wobei die Wasserphase mindestens einen aktiven Wirkstoff aufweist.
Ausführungsform 24. Die Emulsion gemäß einer der vorhergehenden Ausführungsformen, wobei der pH-Wert der Emulsion von 3 bis 8, bevorzugt von 3,5 bis 7,5, insbesondere von 4 bis 7, beträgt.
Ausführungsform 25. Kosmetische Zusammensetzung enthaltend die Emulsion gemäß einer der vorhergehenden Ausführungsformen.
Ausführungsform 26. Sonnenschutzmittel enthaltend mindestens einen UV-Filter und Ligninpartikel, wobei die Ligninpartikel einen mittleren hydrodynamischen Durchmesser von weniger als 800 nm aufweisen.
Ausführungsform 27. Das Sonnenschutzmittel gemäß der vorhergehenden Ausführungsform, wobei die Ligninpartikel einen mittleren hydrodynamischen Durchmesser von weniger als 750 nm, bevorzugt weniger als 700 nm, mehr bevorzugt weniger als 650 nm, mehr bevorzugt weniger als 600 nm, mehr bevorzugt weniger als 550 nm, mehr bevorzugt weniger als 500 nm aufweisen.
Ausführungsform 28. Das Sonnenschutzmittel gemäß einer der vorhergehenden Ausführungsformen, wobei die Ligninpartikel einen mittleren hydrodynamischen Durchmesser von 1 bis 800 nm, bevorzugt von 2 bis 750 nm, mehr bevorzugt von 3 bis 700 nm, mehr bevorzugt von 4 bis 650 nm, mehr bevorzugt von 6 bis 600 nm, mehr bevorzugt von 8 bis 575 nm, mehr bevorzugt von 10 bis 550 nm, mehr bevorzugt von 15 bis 525 nm, noch mehr bevorzugt von 20 bis 500 nm aufweisen. Ausführungsform 29. Das Sonnenschutzmittel gemäß einer der vorhergehenden Ausführungsformen, wobei die Ligninpartikel einen Polydispersitätsindex von weniger als 0,50, bevorzugt weniger als 0,45, mehr bevorzugt weniger als 0,40, mehr bevorzugt weniger als 0,35, mehr bevorzugt weniger als 0,30, mehr bevorzugt weniger als 0,25, mehr bevorzugt weniger als 0,20.
Ausführungsform 30. Das Sonnenschutzmittel gemäß einer der vorhergehenden Ausführungsformen, wobei die Ligninpartikel einen Polydispersitätsindex von 0,01 bis 0,50, bevorzugt von 0,05 bis 0,40, mehr bevorzugt von 0,10 bis 0,30 aufweisen.
Ausführungsform 31. Das Sonnenschutzmittel gemäß einer der vorhergehenden Ausführungsformen, wobei die Konzentration der Ligninpartikel im Sonnenschutzmittel mindestens 0,1 Gew.-%, bevorzugt mindestens 0,25 Gew.-%, mehr bevorzugt mindestens 0,5 Gew.-%, mehr bevorzugt mindestens 0,75 Gew.-%, mehr bevorzugt mindestens 1,0 Gew.-%, mehr bevorzugt mindestens 1,25 Gew.-%, mehr bevorzugt mindestens 1,5 Gew.-%, mehr bevorzugt mindestens 1,7 Gew.-%, mehr bevorzugt mindestens 1,75 Gew.-%, mehr bevorzugt mindestens 1,8 Gew.-%, mehr bevorzugt mindestens 1,85 Gew.-%, mehr bevorzugt mindestens 1,9 Gew.-%, mehr bevorzugt mindestens 1,95 Gew.-%, mehr bevorzugt mindestens 2,0 Gew.-%, mehr bevorzugt mindestens 2,05 Gew.-%, mehr bevorzugt mindestens 2,1 Gew.-%, mehr bevorzugt mindestens 2,15 Gew.-%, mehr bevorzugt mindestens 2,2 Gew.-%, mehr bevorzugt mindestens 2,25 Gew.-%, mehr bevorzugt mindestens 2,3 Gew.-%, mehr bevorzugt mindestens 2,5 Gew.-%, mehr bevorzugt mindestens 2,7 Gew.-%, noch mehr bevorzugt mindestens 3,0 Gew.-% beträgt.
Ausführungsform 32. Das Sonnenschutzmittel gemäß einer der vorhergehenden Ausführungsformen, wobei die Konzentration der Ligninpartikel im Sonnenschutzmittel weniger als 40 Gew.-%, bevorzugt weniger als 30 Gew.-%, mehr bevorzugt weniger als 20 Gew.-%, mehr bevorzugt weniger als 15 Gew.-%, mehr bevorzugt weniger als 10 Gew.-%, mehr bevorzugt weniger als 8 Gew.-%, mehr bevorzugt weniger als 7 Gew.-%, noch mehr bevorzugt weniger als 6 Gew.-% beträgt.
Ausführungsform 33. Das Sonnenschutzmittel gemäß einer der vorhergehenden Ausführungsformen, wobei die Konzentration der Ligninpartikel im Sonnenschutzmittel im Bereich von 0,1 bis 50 Gew.-%, bevorzugt 0,25 bis 45 Gew.-%, mehr bevorzugt 0,5 bis 40 Gew.-%, mehr bevorzugt von 0,75 bis 37 Gew.-%, mehr bevorzugt von 1,0 bis 34 Gew.-%, mehr bevorzugt von 1,25 bis 32 Gew.-%, mehr bevorzugt von 1,5 bis 32 Gew.-%, mehr bevorzugt von 1,7 bis 30 Gew.-%, mehr bevorzugt von 1,75 bis 28 Gew.-%, mehr bevorzugt von 1,8 bis 26 Gew.-%, mehr bevorzugt von 1,85 bis 24 Gew.-%, mehr bevorzugt von 1,9 bis 22 Gew.-%, mehr bevorzugt von 1,95 bis 20 Gew.-%, mehr bevorzugt von 2,0 bis 18 Gew.-%, mehr bevorzugt von 2,05 bis 16 Gew.-%, mehr bevorzugt von 2,1 bis 14 Gew.-%, mehr bevorzugt von 2,15 bis 12 Gew.-%, mehr bevorzugt von 2,2 bis 10 Gew.-%, mehr bevorzugt von 2,25 bis 9 Gew.-%, mehr bevorzugt von 2,3 bis 8 Gew.-%, mehr bevorzugt von 2,5 bis 7 Gew.-%, mehr bevorzugt von 2,7 bis 6 Gew.-%, noch mehr bevorzugt von 3,0 bis 5 Gew.-% beträgt.
Ausführungsform 34. Das Sonnenschutzmittel gemäß einer der vorhergehenden Ausführungsformen, wobei die Konzentration des mindestens einen UV-Filters im Sonnenschutzmittel im Bereich von 1 bis 35 Gew.-%, bevorzugt von 1,5 bis 30 Gew.-%, insbesondere von 2,5 bis 25 Gew.-%, liegt.
Ausführungsform 35. Das Sonnenschutzmittel gemäß der vorhergehenden Ausführungsform, wobei der UV-Filter ausgewählt ist aus der Liste der in kosmetischen Mitteln zugelassenen UV-Filter gemäß Anhang VI Verordnung (EG) Nr. 1223/2009 des Europäischen Parlaments und des Rates vom 30. November 2009 über kosmetische Mittel (konsolidierter Text vom 06.10.2022, European Legislation Identifier (ELI): http://data.europa.eu/eli/reg/2009/1223/2022-10-06).
Ausführungsform 36. Das Sonnenschutzmittel gemäß einer der vorhergehenden Ausführungsformen, wobei der mindestens eine UV-Filter ein anorganischer UV-Filter ist.
Ausführungsform 37. Das Sonnenschutzmittel gemäß der vorhergehenden Ausführungsform, wobei der anorganische UV-Filter ausgewählt ist aus der Gruppe bestehend aus Zinkoxid und/oder Titanium Dioxid.
Ausführungsform 38. Das Sonnenschutzmittel gemäß einer der vorhergehenden Ausführungsformen, wobei der mindestens eine UV-Filter ein anorganischer UV-Filter ist und wobei die Konzentration der Ligninpartikel im Sonnenschutzmittel nicht mehr als 7 Gew.-%, vorzugsweise nicht mehr als 6 Gew.-%, insbesondere nicht mehr als 5 Gew.-% beträgt.
Ausführungsform 39. Das Sonnenschutzmittel gemäß einer der vorhergehenden Ausführungsformen, wobei der mindestens eine UV-Filter ein organischer UV-Filter ist.
Ausführungsform 40. Das Sonnenschutzmittel gemäß einer der vorhergehenden Ausführungsformen, wobei der mindestens eine UV-Filter ein organischer UV-Filter ist und wobei die Konzentration der Ligninpartikel im Sonnenschutzmittel mindestens 1,7 Gew.-%, bevorzugt mindestens 2,0 Gew.-%, noch mehr bevorzugt mindestens 2,3 Gew.-%, noch mehr bevorzugt mindestens 2,6 Gew.-%, noch mehr bevorzugt mindestens 2,9 Gew.-%, noch mehr bevorzugt mindestens 3,2 Gew.-%, noch mehr bevorzugt mindestens 3,5 Gew.-%, noch mehr bevorzugt mindestens 3,8 Gew.-%, noch mehr bevorzugt mindestens 4,1 Gew.-%, noch mehr bevorzugt mindestens 4,4 Gew.-%, noch mehr bevorzugt mindestens 4,7 Gew.-%, noch mehr bevorzugt mindestens 5,0 Gew.-%, noch mehr bevorzugt mindestens 5,3 Gew.-%, noch mehr bevorzugt mindestens 5,6 Gew.-%, noch mehr bevorzugt mindestens 5,9 Gew.-%, noch mehr bevorzugt mindestens 6,2 Gew.-%, noch mehr bevorzugt mindestens 6,5 Gew.-% beträgt.
Ausführungsform 41. Das Sonnenschutzmittel gemäß einer der vorhergehenden Ausführungsformen, wobei der mindestens eine UV-Filter ein organischer UV-Filter ist und wobei die Konzentration der Ligninpartikel im Sonnenschutzmittel nicht mehr als 10 Gew.-%, bevorzugt nicht mehr als 9 Gew.-%, mehr bevorzugt nicht mehr als 8 Gew.-%, insbesondere nicht mehr als 7 Gew.-% beträgt.
Ausführungsform 42. Das Sonnenschutzmittel gemäß einer der vorhergehenden Ausführungsformen, wobei das Sonnenschutzmittel ein Fett und/oder ein Öl enthält und wobei das Fett und/oder das Öl einen SPF-Faktor von mindestens 0,1, bevorzugt mindestens 0,2, mehr bevorzugt mindestens 0,3, mehr bevorzugt mindestens 0,4, mehr bevorzugt mindestens 0,5, mehr bevorzugt mindestens 0,7, mehr bevorzugt mindestens 1,0, mehr bevorzugt mindestens 2,0, aufweist.
Ausführungsform 43. Das Sonnenschutzmittel gemäß einer der vorhergehenden Ausführungsformen, wobei das Fett und/oder das Öl einen SPF-Faktor von 0,1 bis 20, bevorzugt von 0,2 bis 16, mehr bevorzugt 0,3 bis 14, mehr bevorzugt 0,4 bis 12, mehr bevorzugt 0,5 bis 11, mehr bevorzugt 0,6 bis 10, mehr bevorzugt 0,7 bis 9, mehr bevorzugt 0,8 bis 8, aufweist.
Ausführungsform 44. Das Sonnenschutzmittel gemäß einer der vorhergehenden Ausführungsformen, wobei das Fett und/oder das Öl ausgewählt ist aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl, Weizenkeimöl, Walnussöl, Baumwollsamenöl und/oder Mischungen davon; insbesondere Shea Butter.
Ausführungsform 45. Das Sonnenschutzmittel gemäß einer der vorhergehenden Ausführungsformen, wobei das Sonnenschutzmittel ein Fett und/oder ein Öl ausgewählt aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl, Weizenkeimöl, Walnussöl, Baumwollsamenöl und/oder Mischungen davon aufweist; insbesondere Shea Butter.
Ausführungsform 46. Das Sonnenschutzmittel gemäß einer der vorhergehenden Ausführungsformen, wobei die Konzentration des Fetts und/oder des Öls im Sonnenschutzmittel mehr als 10 Gew.-%, bevorzugt mehr als 15 Gew.-%, mehr bevorzugt mehr als 20 Gew.-%, noch mehr bevorzugt mehr als 30 Gew.-%, noch mehr bevorzugt mehr als 40 Gew.-%, noch mehr bevorzugt mehr als 50 Gew.-%, noch mehr bevorzugt mehr als 60 Gew.-%, noch mehr bevorzugt mehr als 70 Gew.-% beträgt.
Ausführungsform 47. Das Sonnenschutzmittel gemäß einer der vorhergehenden Ausführungsformen, wobei die Konzentration des Fetts und/oder des Öls im Sonnenschutzmittel von 10 bis 90 Gew.-%, bevorzugt von 15 bis 80 Gew.-%, noch mehr bevorzugt von 20 bis 70 Gew.-%, noch mehr bevorzugt von 25 bis 60 Gew.-% beträgt.
Ausführungsform 48. Das Sonnenschutzmittel gemäß einer der vorhergehenden Ausführungsformen, wobei das Sonnenschutzmittel eine Sonnencreme, eine Sonnenlotion, ein Sonnengel, ein Lippenbalsam, ein Spray, ein Öl, ein Tonic und/oder eine Milch ist.
Ausführungsform 49. Das Sonnenschutzmittel gemäß einer der vorhergehenden Ausführungsformen, wobei das Sonnenschutzmittel die Emulsion nach einer der vorhergehenden Ausführungsformen aufweist.
Ausführungsform 50. Das Sonnenschutzmittel gemäß einer der vorhergehenden Ausführungsformen, wobei die Ligninpartikel so sind wie in einer der vorhergehenden Ausführungsformen betreffend die Emulsion definiert.

Die vorliegende Erfindung wird durch die folgenden Beispiele und die Figuren illustriert, auf welche sie selbstverständlich nicht eingeschränkt ist.
**Figur 1.** (A) Mittlerer hydrodynamischer Durchmesser der Ligninpartkel in Abhängigkeit der Strömungsgeschwindigkeit während der Fällung. (B) Polydispersitätsindex der Ligninpartikel in Abhängigkeit der Strömungsgeschwindigkeit während der Fällung.
**Figur 2****.** Fluoreszenzmikroskopie-Aufnahme einer Emulsion enthaltend Ligninpartikel mit einem mittleren hydrodynamischen Durchmesser von 80 nm und einen Polydispersitätsindex von 0,21. Das fluoreszierende Lignin lagert sich an der Grenzfläche zwischen Öl- und Wasser an.
**Figur 3.** (A) Vergleich der unbehandelten Suspension (links) und der 50°C temperaturbehandelten Suspension (rechts) nach 10s in senkrechter Position. (B) Vergleich der unbehandelten Emulsion (links) und der 50°C temperaturbehandelten Emulsion (rechts) nach 10 s in senkrechter Position.
**Figur 4.** (A) Elektronenmikroskop Aufnahmen von Rohlignin (oben) und nach Beispiel 1 hergestellten Ligninpartikeln (unten). (B) SPF Werte von Emulsionen ohne Lignin, mit Rohlignin und mit nach Beispiel 1 hergestellten Ligninpartikeln.
**Figur 5****.** SPF Werte von Sonnencremeformulierungen mit verschiedenen Typen von UV-Filtern und in Kombination mit und ohne Ligninpartikel.
**Figur 6****.** SPF Werte einer Formulierung enthaltend Shea Butter mit und ohne Ligninpartikeln. Der SPF-Wert der Formulierung mit Shea Butter wurde durch den Zusatz von Ligninpartikeln deutlich verstärkt.

### Beispiel 1. Herstellung und Charakterisierung von Ligninpartikeln

### Herstellung

Die Herstellung von Ligninpartikeln wurde im Wesentlichen so durchgeführt wie beschrieben in WO 2020/000008 A1 und aus Beisl et al. (Molecules 23 (2018), 633-646) . Ein durchgeführtes Herstellungsverfahren ist in der Folge beispielhaft beschrieben:
Rohlignin (Organosolv Lignin der Fa. Chemical Point) wurde mit einer Beladung von 9 g/l in einer wässrigen 60 Gew.-% EtOH Lösung gelöst. Unlösliche Bestandteile wurden nach 12h Lösungszeit mit einem Filter (5µm Porengröße) abgetrennt. Die partikelfreie Lösung wurde in einem kontinuierlichem T-Mischer (Eingang Ligninlösung 4mm Innendurchmesser, Eingang Wasser 8mm Innendurchmesser, Ausgang Suspension 10mm Innendurchmesser) in einem Volumenverhältnis von 1:3 (Ligninlösung:Wasser) bei unten angegebenen Strömungsgeschwindigkeiten in der Ausgangsleitung gemischt.

### Messung der Partikelgrößenverteilung

Der Hydrodynamische Durchmesser und Polydispersitätsindex (PDI) wurde nach Verdünnung mit Wasser (20µl Partikelsupension mit 3ml Wasser) in einem Litesizer 500 von Anton Paar gemessen. Der Brechungsindezes der Partikel wurde mit 1,53 and Absorptionskoeffizent mit 0,1 1/m in der Messsoftware eingestellt.

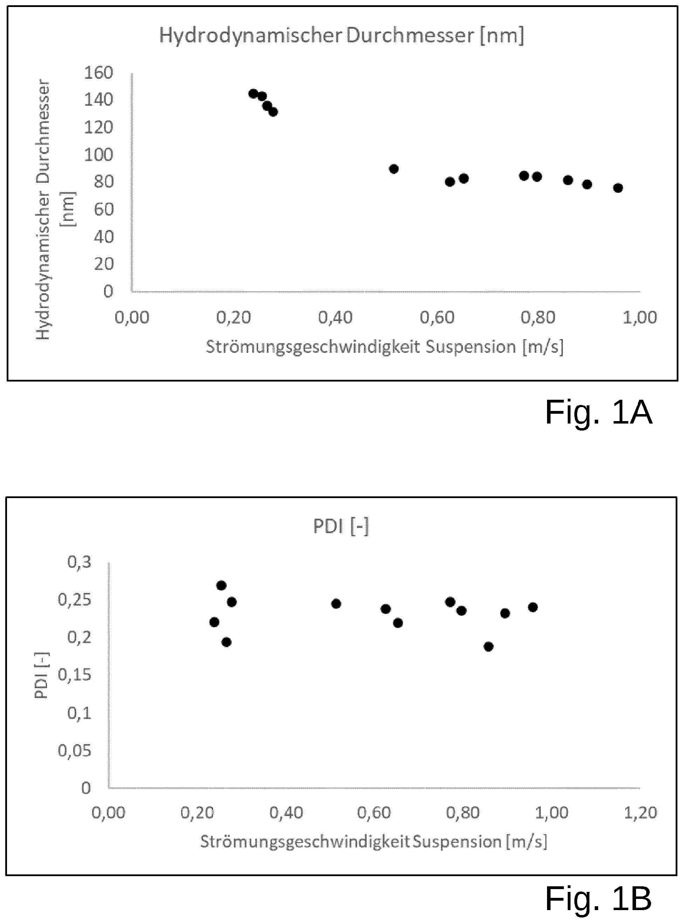

Der Hydrodynamische Durchmesser und der Polydispersiätsindex der Ligninpartikel wurden in Abhängigkeit der Strömungsgeschwindigkeit in der Fälleinrichtung untersucht. Im untersuchten Bereich konnte ein sinkender hydrodynamischer Durchmesser mit zunehmender Strömungsgeschwindigkeit festgestellt werden. Betrachtet man den Polydisperistätsindex zeigte sich hier kein Einfluss. Bei allen betrachteten Strömungsgeschwindigkeiten zeigte sich ein ähnlicher PDI. Die Resultate des gemessenen mittleren hydrodynamischen Durchmessers und des PDI sind in Figur 1 gezeigt.

### Beispiel 2. Herstellung von Emulsionen.

Emulsionen bestehen jeweils aus einer Öl- und einer Wasserphase. Die Ölphase wurde separat angemischt und auf 80°C erwärmt und vor der Vereinigung mit der Wasserphase auf 50°C abgekühlt. Die Ligninpartikel wurden in die Wasserphase inkorporiert. Die genauen Zusammensetzungen der Öl- und Wasserphasen werden in den nachfolgenden Beispielen angegeben.

Sobald die Ölphase auf eine Temperatur von 50°C abgekühlt war, wurde sie mit einem Homogenisator (Ultra Turrax T25 mit einem S 25 KD - 25G Aufsatz) bei 8000 Umdrehungen pro Minute homogenisiert. Währenddessen wurde die Wasserphase langsam zur Ölphase hinzugegeben. Die Homogenisierung wurde so lange durchgeführt, bis eine homogene Emulsion entstanden war.

### Beispiel 3. Verhalten von Ligninpartikel in Emulsionen

Um die Wirkweise der Ligninpartikel als Emulgator näher zu untersuchen, wurde eine Emulsion unter einem Fluoreszenzmikroskop untersucht. Lignin fluoresziert, weshalb die Position der Ligninpartikel innerhalb der Emulsion identifiziert werden konnte.

Die Ölphase bestand in diesem Versuch aus 50g MCT Öl (INCI Caprylic/Capric Triglyceride), die Wasserphase aus 50g Wasser mit 4,85 Gew.-% Ligninpartikeln. Die verwendeten Partikel wiesen einen mittleren hydrodynamischen Durchmesser von 80 nm und einen Polydispersitätsindex von 0,21 auf. Die Ölphase wurde bei Raumtemperatur langsam zur Wasserphase hinzugegeben, während diese mit einem Homogenisator (Ultra Turrax T25 mit einem S 25 KD - 25G Aufsatz) bei 12000 Umdrehungen pro Minute homogenisiert wurde.

Die Entstandene Emulsion wurde mit einem Fluoreszenzmikroskop (Nikon Upright Eclipse Ci, Objektiv Plan Apo λ 40×/0.95 mit einem Nikon LV-UEPI2 Universal Epi Illuminator 2) untersucht und mit einer Wellenlänge von 465-495 nm angeregt und bei 515-555 nm gemessen. Die Emulsion wurde auf einem Mikroskop Glas platziert und mit einem Deckglas mit einem Abstandhalter bedeckt.

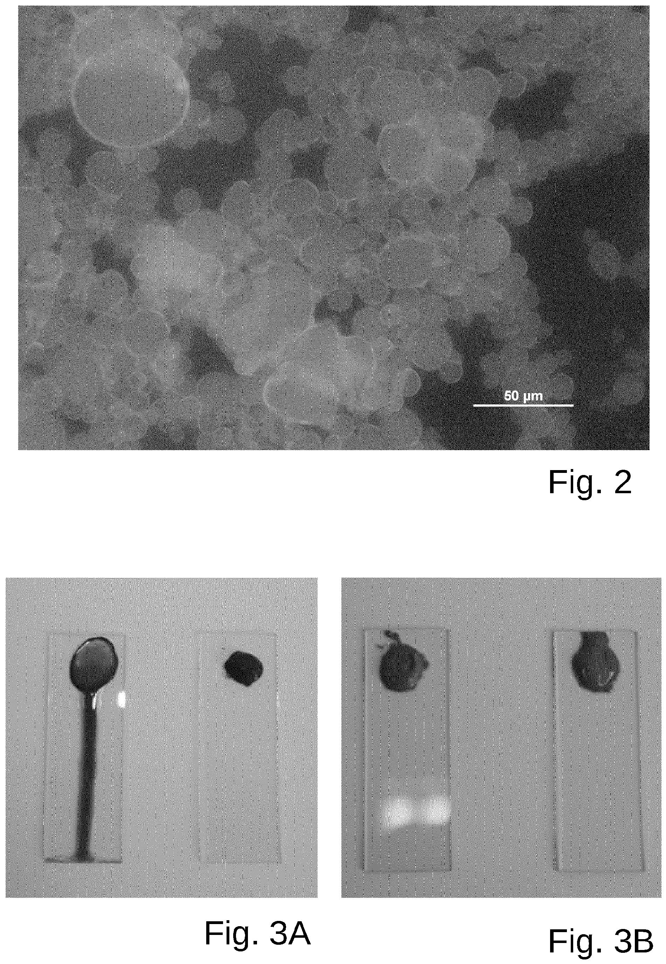

In Figur 2 sind die Öltropfen zu erkennen, welche an ihren Rändern eine erhöhte Fluoreszenz (hellere Bereiche) erkennen lassen während in der Wasserphase keine Fluoreszenz zu erkennen ist. Dies deutet darauf hin, dass sich die Ligninpartikel an der Grenzfläche zwischen Öl- und Wasser anlagern und dadurch die Emulsion stabilisieren.

### Beispiel 4. Stabilität von Ligninpartikeln in Emulsionen.

Um das Temperaturverhalten von Ligninpartikel-Suspensionen und Ligninpartikel-basierten Emulsionen zu vergleichen, wurden die Suspension und Emulsion jeweils für 30min verschlossen in einem Probenbehälter in einem Wasserbad bei 50°C behandelt.

Die Suspension enthielt 15,4 Gew.-% Ligninpartikel und die Emulsion wurde basierend auf der Methode in Beispiel 2 hergestellt. Die Ölphase bestand aus 17,5g Shea Butter (INCI: BUTYROSPERMUM PARKII BUTTER), 17,5g Babassu Öl (INCI: Orbignya Oleifera Seed Oil) und 15g Hallbrite EZ FLO ZDX. Die Wasserphase hatte eine Gesamtmasse von 50g und eine Ligninpartikel-Konzentration von 4,85 Gew.-%.

Nach der beschriebenen Temperaturbehandlung wurden die Emulsionen und Suspensionen auf einen Mikroskop Träger aus Glas aufgegeben und für 10s in senkrechter Position gehalten. Die Temperaturbehandlung der Suspension zeigte sehr großen Einfluss auf deren Fließverhalten, welches sich durch die Behandlung wesentlich verschlechterte, da die Suspension viskoser wurde (siehe Figur 3A). Das Fließverhalten der temperaturbehandelten Emulsion zeigte hingegen kaum einen Unterschied zur unbehandelten Emulsion (siehe Figur 3B). Damit wurde gezeigt, dass die negative Auswirkung erhöhter Temperatureinwirkung auf das Fließverhalten der Ligninpartikel-Suspension durch die Einarbeitung der Ligninpartikel-Suspension in Emulsionen aufgehoben wird.

### Beispiel 5. "SPF-Booster" Effekt

Die Wirkung von kolloidalen Ligninpartikeln wie im Zusammenhang mit der Erfindung definiert auf den Lichtschutzfaktor (SPF-Wert) von Sonnenschutzmitteln wurde mit der Wirkung von nicht-kolloidalem Rohlignin verglichen.

Die Ligninpartikel wurde im Wesentlichen hergestellt, wie in Beispiel 1 beschrieben. Die so erhaltenen Partikel wiesen einen mittleren hydrodynamischen Durchmesser von 108 nm und einen Polydispersitätsindex von 0.24 auf. Zum Vergleich wurden Emulsionen mit diesen Ligninpartikeln, mit nicht-kolloidalem Rohlignin und gänzlich ohne Lignin hergestellt.

Die Emulsionen wurden hergestellt wie in Beispiel 2 beschrieben. Die Ölphase bestand aus 17,5g Shea Butter (INCI: BUTY-ROSPERMUM PARKII BUTTER), 17,5g Babassu Öl (INCI: Orbignya Oleifera Seed Oil) und 15g Hallbrite EZ FLO ZDX (UV-Filter). Die Wasserphase hatte eine Gesamtmasse von 50g und enthielt entweder kein Lignin oder eine Ligninpartikel- bzw. Rohlignin-Konzentration von 4,85 Gew.-%.

Der SPF Wert der Emulsionen wurde wie in Beispiel 6 beschrieben gemessen.

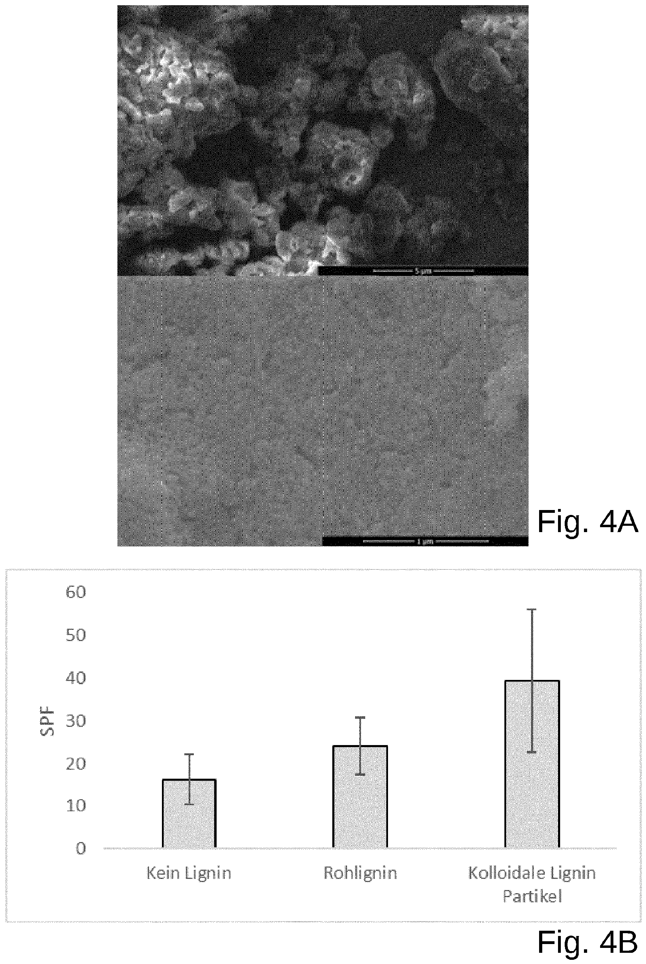

Um die mikroskopischen Strukturunterschiede von Rohlignin und Ligninpartikel zu untersuchen, wurden Aufnahmen mit einem Elektronenmikroskop erstellt. Es war klar ersichtlich, dass die nach Beispiel 1 hergestellten Ligninpartikel wesentlich kleiner und gleichmäßiger waren als die Partikel des Rohlignins (siehe Figur 4A) .

Der SPF Wert der Emulsionen ohne Lignin konnte sowohl durch die Zugabe von Ligninpartikeln als auch Rohlignin gesteigert werden. Die nach Beispiel 1 hergestellten Ligninpartikel konnten den SPF jedoch im Vergleich zum Rohlignin signifikant stärker erhöhen (siehe Figur 4B).

### Beispiel 6. Konzentrationsabhängigkeit des "SPF-Booster" Effekts

Um die Abhängigkeit des "SPF-Booster" Effekts von der Konzentration der Ligninpartikel nachzuweisen, wurden Sonnencremeformulierungen mit verschiedenen Ligninpartikel-Konzentrationen hergestellt. Die Sonnencremeformulierungen bestanden jeweils aus einer Öl- und einer Wasserphase. Die Ölphase war in allen 4 Sonnencremeformulierungen ident und bestand aus 17,5g Shea Butter (INCI: BUTYROSPERMUM PARKII BUTTER), 17,5g Babassu Öl (INCI: Orbignya Oleifera Seed Oil) und 15g Hallbrite EZ FLO ZDX (UV-Filter) . Die Ölphase wurde auf 80°C erwärmt und vor der Vereinigung mit der Wasserphase auf 50°C abgekühlt. Die Wasserphase hatte eine Gesamtmasse von 50g und enthielt die Ligninpartikel in der erforderlichen Konzentration, um die gewünschte finale Ligninpartikel-Konzentration in der finalen Formulierung erreichen zu können.

Die Ölphase wurde nach Abkühlen auf eine Temperatur von 50°C mit einem Homogenisator (Ultra Turrax T25 mit einem S 25 KD - 25G Aufsatz) bei 8000 Umdrehungen pro Minute homogenisiert. Währenddessen wurde die Wasserphase mit Raumtemperatur langsam zur Ölphase hinzugegeben. Die Homogenisierung wurde fortgesetzt, bis eine homogene Emulsion entstanden war.

Die erhaltenen Sonnencremeformulierungen wurden für 24 Stunden bei Raumtemperatur gelagert und danach der in-vitro SPF bestimmt. Dafür wurden Blendterm Fixierpflaster (Hersteller 3M) auf Quarzglas Mikroskop Trägern aufgeklebt und die Oberflächen mit jeweils 2 mg/cm³ der Sonnencremeformulierungen manuell bestrichen. In einem UV-Vis Spektrometer (UV-1800, Shimadzu) wurde die Absorption der Proben in einem Wellenlängenbereich von 190-600nm in Stufen von 1 nm gemessen. Die Proben wurden dabei direkt vor dem Detektor platziert und ein leeres Blendterm Fixierpflaster auf einem Quarzglas Mikroskop Träger als Referenz verwendet. Zur Berechnung des SPF wurden die Absorptionswerte im Bereich von 290 - 400 nm herangezogen. Das Spektrum der UV Strahlungsquelle (laut DIN EN ISO 24443:2020-06 für SPF Prüfung: UV-SSR-Bestrahlungsstärken (DIN EN ISO 24443:2020-06 Anhang C)) wurde bei jeder Wellenlänge mit dem entsprechenden Empfindlichkeitswert des Erythemwirksamkeitsspektrums (Norm Anhang C) multipliziert. Durch Integration im genannten Wellenlängenbereich (290 - 400 nm) wurde die resultierende erythemwirksame Bestrahlungsstärke erhalten. Um die durch die Sonnencremeformulierungen hindurchgelassene erythemwirksame Bestrahlungsstärke zu berechnen, wurden die Transmissionswerte der Messungen im Spektrometer bei jeder Wellenlänge mit der jeweiligen erythemwirksamen Bestrahlungsstärke multipliziert und im Wellenlängenbereich (290- 400 nm) integriert. Der in-vitro bestimmte SPF wurde mit dem Verhältnis dieser Integrale berechnet. Es wurden jeweils 10 Einzelmessungen durchgeführt und mit Hilfe der 1,5*IQR Regel die Ausreißer bestimmt. Danach wurde jeweils ein Mittelwert der SPF Ergebnisse berechnet.

Der SPF Boost wurde jeweils als prozentuale Erhöhung des SPF im Vergleich zur Formulierung ohne Lignin spezifiziert.

**Tabelle 1:**

| **SPF** | **Gew.-% Ligninpartikel in der finalen Formulierung** | **SPF boost** |
|---|---|---|
| 36,02 | 2,5 | 187% |
| 18,13 | 1,7 | 44% |
| 17,33 | 1 | 38% |
| 12,56 | 0 | 0% |

Die Ergebnisse zeigten bereits einen SPF Boost bei einer Zugabe von 1 Gew.-% Ligninpartikel. Ab einer Einsatzkonzentration von 1,7 Gew.-% Ligninpartikel wurde jedoch ein sprunghafter Anstieg des SPF Boosts auf bis zu 187% bei einer Ligninpartikel-Konzentration von 2,5 Gew.-% festgestellt.

### Beispiel 7. "SPF-Booster" Effekt mit unterschiedlichen UV-Filter

Um den "SPF-Booster" Effekt von Ligninpartikeln in Kombination mit organischen und anorganischen UV-Filtern zu testen, wurden Emulsionen nach der in Beispiel 2 angegebenen Methode und mit den nachfolgenden Zusammensetzungen hergestellt. Die Ligninpartikel-Konzentration in den Emulsionen betrug jeweils 2,43 Gew.-%.

**Tabelle 2:**

| **Emulsions-Variante** | **Zusammensetzung Ölphase** | | **Zusammensetzung Wasserphase** | |
|---|---|---|---|---|
| TiO₂ Nano | A15-TiO2-SX-NJE8 | 9 g | Ligninsupsension oder Wasser | 50 g |
| | Shea butter | 20,5 g | | |
| | Babassu oil | 20,5 g | | |
| Organische Filter | Eusolex 9020 (b) | 2 g | Ligninsupsension oder Was-ser | 50 g |
| | Eusolex OCR (a) | 6 g | | |
| | Shea butter (a) | 21 g | | |
| | Babassu oil (a) | 21 g | | |
| ZnO | Hallbrite ZDX Flo | 15 g | Ligninsupsension oder Wasser | 50 g |
| | Shea butter | 17,5 g | | |
| | Babassu oil | 17,5 g | | |
| ZnO + Organische Filter | Hallbrite ZDX Flo Plus | 15 g | Ligninsupsension oder Wasser | 50 g |
| | Shea butter | 17,5 g | | |
| | Babassu oil | 17,5 g | | |
| Ohne UV-Filter | Shea butter | 25 g | Ligninsupsension oder Wasser | 50 g |
| | Babassu oil | 25 g | | |

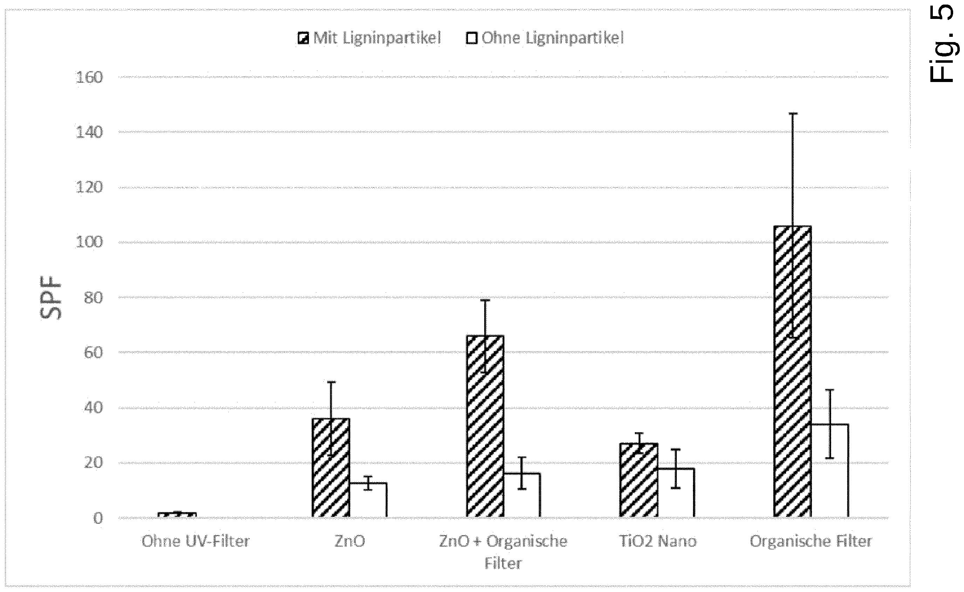

Der SPF der Emulsionen wurden mit der in Beispiel 5 genannten Methode bestimmt. Es konnte eine signifikante Erhöhung des SPF durch Ligninpartikel mit allen getesteten UV-Filtern erreicht werden (siehe Figur 5). Dies bestätigt den "SPF-Booster" Effekt mit organischen und anorganischen UV-Fitern.

### Beispiel 8. Synergie von Ligninpartikeln mit UV-absorbierenden Ölen und/oder Fetten

Um die Synergie von Lignin Partikeln in Kombination mit UV-absorbierenden Öl- und Fetten zu untersuchen, wurden Formulierung mit und ohne Ligninpartikel hergestellt und deren SPF gemessen. Als Basis wurde Shea Butter ausgewählt, da diese ohne Zusätze schon einen geringen SPF aufweist.

Die Emulsionen wurden hergestellt wie in Beispiel 2 beschrieben. Die Ölphase bestand aus 25g Shea Butter (INCI: BUTY-ROSPERMUM PARKII BUTTER), und 25g Babassu Öl (INCI: Orbignya Oleifera Seed Oil). Die Wasserphase hatte eine Gesamtmasse von 50g und enthielt entweder eine Ligninpartikelkonzentration von 4,85 Gew.-% oder keine Ligninpartikel. Die verwendeten Partikel wiesen einen mittleren hydrodynamischen Durchmesser von 350 nm und einen Polydispersitätsindex von 0,25 auf.

Die erhaltenen kosmetischen Formulierungen wurden für 24 Stunden bei Raumtemperatur gelagert und danach der in-vitro SPF bestimmt. Dafür wurden Quarzglas Mikroskop Träger mit jeweils 2 mg/cm3 der Sonnencremeformulierungen manuell bestrichen. In einem UV-Vis Spektrometer (UV-1800, Shimadzu) wurde die Absorption der Proben in einem Wellenlängenbereich von 190-600nm in Stufen von 1 nm gemessen. Die Proben wurden dabei direkt vor dem Detektor platziert und ein leeres Blendterm Fixierpflaster auf einem Quarzglas Mikroskop Träger als Referenz verwendet. Zur Berechnung des SPF wurden die Absorptionswerte im Bereich von 290 - 400 nm herangezogen. Das Spektrum der UV Strahlungsquelle (laut DIN EN ISO 24443:2020-06 für SPF Prüfung: UV-SSR-Bestrahlungsstärken (DIN EN ISO 24443:2020-06 Anhang C)) wurde bei jeder Wellenlänge mit dem entsprechenden Empfindlichkeitswert des Erythemwirksamkeitsspektrums (Norm Anhang C) multipliziert. Durch Integration im genannten Wellenlängenbereich (290 - 400 nm) wurde die resultierende erythemwirksame Bestrahlungsstärke erhalten. Um die durch die kosmetischen Formulierungen hindurchgelassene erythemwirksame Bestrahlungsstärke zu berechnen, wurden die Transmissionswerte der Messungen im Spektrometer bei jeder Wellenlänge mit der jeweiligen erythemwirksamen Bestrahlungsstärke multipliziert und im Wellenlängenbereich (290 - 400 nm) integriert. Der in-vitro bestimmte SPF wurde mit dem Verhältnis dieser Integrale berechnet. Es wurden jeweils 10 Einzelmessungen durchgeführt und mit Hilfe der 1,5*IQR Regel die Ausreißer bestimmt. Danach wurde jeweils ein Mittelwert der SPF Ergebnisse berechnet.

Figur 6 zeigt die erreichten SPF Ergebnisse der hergestellten Formulierung. Die Formulierung ohne Ligninpartikel erreichte einen SPF von 1,2 und die Formulierung mit Ligninpartikel einen SPF von 3,1. Dies zeigt, dass die Ligninpartikel mit natürlichen Fetten bzw Ölen zusammenwirken und die UV-absorbierende Leistung dadurch steigern werden kann.

## Patentansprüche

1. Emulsion enthaltend Ligninpartikel, wobei die Ligninpartikel einen mittleren hydrodynamischen Durchmesser von weniger als 800 nm und einen Polydispersitätsindex von weniger als 0,50 aufweisen.

2. Die Emulsion gemäß Anspruch 1, wobei die Konzentration der Ligninpartikel in der Emulsion mindestens 1,7 Gew.-%, vorzugsweise mindestens 2,0 Gew.-% beträgt.

3. Die Emulsion gemäß einem der vorhergehenden Ansprüche, wobei die Emulsion eine Fettphase und eine Wasserphase aufweist, wobei die Fettphase ein Fett und/oder ein Öl aufweist und wobei das Fett und/oder das Öl einen SPF-Faktor von mindestens 0,1 aufweist.

4. Die Emulsion gemäß einem der vorhergehenden Ansprüche, wobei die Emulsion ein Fett und/oder ein Öl ausgewählt aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl und/oder Mischungen davon aufweist, insbesondere Shea Butter.

5. Die Emulsion Anspruch 3 oder 4, wobei die Konzentration des Fetts und/oder des Öls in der Emulsion mehr als 10 Gew.-%, bevorzugt mehr als 30 Gew.-%, beträgt.

6. Kosmetische Zusammensetzung enthaltend die Emulsion gemäß einem der vorhergehenden Ansprüche.

7. Sonnenschutzmittel enthaltend mindestens einen UV-Filter und Ligninpartikel, wobei die Ligninpartikel einen mittleren hydrodynamischen Durchmesser von weniger als 800 nm und einen Polydispersitätsindex von weniger als 0,50, vorzugsweise weniger als 0,30, aufweisen.

8. Das Sonnenschutzmittel gemäß einem der vorhergehenden Ansprüche, wobei die Konzentration der Ligninpartikel im Sonnenschutzmittel mindestens 1,7 Gew.-%, vorzugsweise mindestens 2,0 Gew.-% beträgt.

9. Das Sonnenschutzmittel gemäß einem der vorhergehenden Ansprüche, wobei der mindestens eine UV-Filter ein anorganischer UV-Filter ist, vorzugsweise ausgewählt aus der Gruppe bestehend aus Zinkoxid und/oder Titanium Dioxid.

10. Das Sonnenschutzmittel gemäß einem der vorhergehenden Ansprüche, wobei der mindestens eine UV-Filter ein anorganischer UV-Filter ist und wobei die Konzentration der Ligninpartikel im Sonnenschutzmittel nicht mehr als 7 Gew.-%, insbesondere nicht mehr als 5 Gew.-% beträgt.

11. Das Sonnenschutzmittel gemäß einem der vorhergehenden Ansprüche, wobei der mindestens eine UV-Filter ein organischer UV-Filter ist und wobei die Konzentration der Ligninpartikel im Sonnenschutzmittel mindestens 1,7 Gew.-%, bevorzugt mindestens 2,0 Gew.-% beträgt.

12. Das Sonnenschutzmittel gemäß einem der vorhergehenden Ansprüche, wobei das Sonnenschutzmittel ein Fett und/oder ein Öl aufweist und wobei das Fett und/oder das Öl einen SPF-Faktor von mindestens 0,1 aufweist.

13. Das Sonnenschutzmittel gemäß einem der vorhergehenden Ansprüche, wobei das Sonnenschutzmittel ein Fett und/oder ein Öl ausgewählt aus der Gruppe bestehend aus Shea Butter, Jojoba Öl, Karottensamenöl, Sojaöl, Nachtkerzenöl, Olivenöl, Kokosöl, Rizinusöl, Mandelöl, Senföl, Chaulmoograöl, Sesamöl, Pfefferminzöl, Tulsi Öl, Zitronengrasöl, Lavendelöl, Orangenöl, Zitronenöl, Eukalyptusöl, Teebaumöl, Rosenöl, Weizenkeimöl, Walnussöl, Baumwollsamenöl und/oder Mischungen davon aufweist; insbesondere Shea Butter.

14. Das Sonnenschutzmittel gemäß einem der vorhergehenden Ansprüche, wobei das Sonnenschutzmittel eine Sonnencreme, eine Sonnenlotion, ein Sonnengel, ein Lippenbalsam, ein Spray, ein Öl, ein Tonic und/oder eine Milch ist.

15. Das Sonnenschutzmittel gemäß einem der vorhergehenden Ansprüche, wobei das Sonnenschutzmittel die Emulsion nach einem der vorhergehenden Ansprüche aufweist.
